# EUROPEAN PATENT SPECIFICATION

(11) **EP 2 714 058 B1**
(45) Date of publication and mention of the grant of the patent: **19.12.2018**
(21) Application number: 12793424.8
(22) Date of filing: 04.06.2012
(51) Int. Cl.: A61K 35/28, C12N 5/077, C12N 5/0789, A61P 25/14, A61K 38/00, C12N 5/0775

(54) **METHOD OF TREATING THE EFFECTS OF STROKE**
VERFAHREN ZUR BEHANDLUNG DER FOLGEN EINES SCHLAGANFALLS
PROCÉDÉ DE TRAITEMENT DES EFFETS D'UN ACCIDENT VASCULAIRE CÉRÉBRAL

(30) Priority: 03.06.2011 US 201161493057 P
(43) Date of publication of application: 09.04.2014
(73) Proprietor: Mesoblast, Inc., New York, New York 10016 (US); Central Adelaide Local Health Network Inc, Adelaide, South Australia 5000 (AU)
(72) Inventor: KOBLAR, Simon Andrea, Millswood, South Australia 5034 (AU); GRONTHOS, Stan, Colonel Light Gardens, South Australia 5041 (AU); ARTHUR, Agnieszka, Uraidla, South Australia 5142 (AU)
(74) Representative: Schnappauf, Georg
(86) International application number: PCT/AU2012/000630
(87) International publication number: WO 2012/162758

(56) References cited:
- WO-A1-2004/084921
- WO-A1-2008/148105
- WO-A2-2008/036374
- WO-A2-2008/052046
- WO-A2-2011/007348
- BAKONDI BENJAMIN ET AL: "CD133 identifies a human bone marrow stem/progenitor cell sub-population with a repertoire of secreted factors that protect against stroke.", MOLECULAR THERAPY : THE JOURNAL OF THE AMERICAN SOCIETY OF GENE THERAPY NOV 2009, vol. 17, no. 11, November 2009 (2009-11), pages 1938-1947, XP002730928, ISSN: 1525-0024
- GRONTHOS, S. ET AL.: 'Heat Shock Protein-90 beta Is Expressed at the Surface of Multipotential Mesenchymal Precursor Cells: Generation of a Novel Monoclonal Antibody, STRO-4 With Specificity for Mesenchymal Precursor Cells From Human and Ovine Tissues' STEM CELLS AND DEVELOPMENT. vol. 18, no. 9, 2009, pages 1253 - 1262, XP008143973
- BONFIELD, T.L. ET AL.: 'Human Mesenchymal Stem Cells Suppress Chronic Airway Inflammation in the Murine Ovalbumin Asthma Model' AMERICAN JOURNAL OF PHYSIOLOGY-LUNG CELLULAR AND MOLECULAR PHYSIOLOGY vol. 299, 2010, pages L760 - L770, XP055138815

### SEQUENCE LISTING

<110> Meoblast, Inc
<120> METHODS OF TREATING THE EFFECTS OF STROKE
<130> 512140
<150> 61/493057
   <151> 2011-06-03
<160> 36
<170> PatentIn version 3.5
<210> 1
   <211> 20
   <212> DNA
   <213> artificial
<220>
   <223> oligonucleotide for amplifying GAPDH
<400> 1
   cactgacacg ttggcagtgg 20
<210> 2
   <211> 20
   <212> DNA
   <213> artificial
<220>
   <223> oligonucleotide for amplifying GAPDH
<400> 2
   catggagaag gctggggctc 20
<210> 3
   <211> 20
   <212> DNA
   <213> artificial
<220>
   <223> oligonucleotide for amplifying SDF-1
<400> 3
   gagacccgcg ctcgtccgcc 20
<210> 4
   <211> 21
   <212> DNA
   <213> artificial
<220>
   <223> oligonucleotide for amplifying SDF-1
<400> 4
   gctggactcc tactgtaagg g 21
<210> 5
   <211> 22
   <212> DNA
   <213> artificial
<220>
   <223> oligonucleotide for amplifying IL-lbeta
<400> 5
   aggaagatgc tggttccctc tc 22
<210> 6
   <211> 23
   <212> DNA
   <213> artificial
<220>
   <223> oligonucleotide for amplifying IL-lbeta
<400> 6
   cagttcagtg atcgtacagg tgc 23
<210> 7
   <211> 29
   <212> DNA
   <213> artificial
<220>
   <223> oligonucleotide for amplifying FLT-1
<400> 7
   tcactatgga agatctgatt tcttacagt 29
<210> 8
   <211> 25
   <212> DNA
   <213> artificial
<220>
   <223> oligonucleotide for amplifying FLT-1
<400> 8
   ggtataaata cacatgtgct tctag 25
<210> 9
   <211> 20
   <212> DNA
   <213> artificial
<220>
   <223> oligonucleotide for amplifying TNFalpha
<400> 9
   tcagatcatc ttctcgaacc 20
<210> 10
   <211> 20
   <212> DNA
   <213> artificial
<220>
   <223> oligonucleotide for amplifying TNFalpha
<400> 10
   cagatagatg ggctcatacc 20
<210> 11
   <211> 20
   <212> DNA
   <213> artificial
<220>
   <223> oligonucleotide for amplifying KDR
<400> 11
   tatagatggt gtaacccgga 20
<210> 12
   <211> 21
   <212> DNA
   <213> artificial
<220>
   <223> oligonucleotide for amplifying KDR
<400> 12
   tttgtcactg agacagcttg g 21
<210> 13
   <211> 21
   <212> DNA
   <213> artificial
<220>
   <223> oligonucleotide for amplifying RANK-ligand
<400> 13
   aacaggcctt tcaaggagct g 21
<210> 14
   <211> 22
   <212> DNA
   <213> artificial
<220>
   <223> oligonucleotide for amplifying RANK-ligand
<400> 14
   taaggagggg ttggagacct cg 22
<210> 15
   <211> 20
   <212> DNA
   <213> artificial
<220>
   <223> oligonucleotide for amplifying Leptin
<400> 15
   atgcattggg aaccctgtgc 20
<210> 16
   <211> 20
   <212> DNA
   <213> artificial
<220>
   <223> oligonucleotide for amplifying Leptin
<400> 16
   gcacccaggg ctgaggtcca 20
<210> 17
   <211> 21
   <212> DNA
   <213> artificial
<220>
   <223> oligonucleotide for amplifying CBFA-1
<400> 17
   gtggacgagg caagagtttc a 21
<210> 18
   <211> 21
   <212> DNA
   <213> artificial
<220>
   <223> oligonucleotide for amplifying CBFA-1
<400> 18
   tggcaggtag gtgtggtagt g 21
<210> 19
   <211> 28
   <212> DNA
   <213> artificial
<220>
   <223> oligonucleotide for amplifying PPARgamma2
<400> 19
   aactgcgggg aaacttggga gattctcc 28
<210> 20
   <211> 26
   <212> DNA
   <213> artificial
<220>
   <223> oligonucleotide for amplifying PPARgamma2
<400> 20
   aataataagg tggagatgca ggctcc 26
<210> 21
   <211> 21
   <212> DNA
   <213> artificial
<220>
   <223> oligonucleotide for amplifying OCN
<400> 21
   atgagagccc tcacactcct c 21
<210> 22
   <211> 19
   <212> DNA
   <213> artificial
<220>
   <223> oligonucleotide for amplifying OCN
<400> 22
   cgtagaagcg ccgataggc 19
<210> 23
   <211> 20
   <212> DNA
   <213> artificial
<220>
   <223> oligonucleotide for amplifying MyoD
<400> 23
   aagcgccatc tcttgaggta 20
<210> 24
   <211> 20
   <212> DNA
   <213> artificial
<220>
   <223> oligonucleotide for amplifying MyoD
<400> 24
   gcgagaaacg tgaacctagc 20
<210> 25
   <211> 20
   <212> DNA
   <213> artificial
<220>
   <223> oligonucleotide for amplifying SMMHC
<400> 25
   ctgggcaacg tagtaaaacc 20
<210> 26
   <211> 20
   <212> DNA
   <213> artificial
<220>
   <223> oligonucleotide for amplifying SMMHC
<400> 26
   tatagctcat tgcagcctcg 20
<210> 27
   <211> 21
   <212> DNA
   <213> artificial
<220>
   <223> oligonucleotide for amplifying GFAP
<400> 27
   ctgttgccag agatggaggt t 21
<210> 28
   <211> 20
   <212> DNA
   <213> artificial
<220>
   <223> oligonucleotide for amplifying GFAP
<400> 28
   tcatcgctca ggaggtcctt 20
<210> 29
   <211> 24
   <212> DNA
   <213> artificial
<220>
   <223> oligonucleotide for amplifying nestin
<400> 29
   ggcagcgttg gaacagaggt tgga 24
<210> 30
   <211> 24
   <212> DNA
   <213> artificial
<220>
   <223> oligonucleotide for amplifying nestin
<400> 30
   ctctaaactg gagtggtcag ggct 24
<210> 31
   <211> 21
   <212> DNA
   <213> artificial
<220>
   <223> oligonucleotide for amplifying SOX9
<400> 31
   ctctgcctgt ttggactttg t 21
<210> 32
   <211> 21
   <212> DNA
   <213> artificial
<220>
   <223> oligonucleotide for amplifying SOX9
<400> 32
   cctttgcttg ccttttacct c 21
<210> 33
   <211> 20
   <212> DNA
   <213> artificial
<220>
   <223> oligonucleotide for amplifying collagen type X
<400> 33
   agccagggtt gccaggacca 20
<210> 34
   <211> 20
   <212> DNA
   <213> artificial
<220>
   <223> oligonucleotide for amplifying collagen type X
<400> 34
   ttttcccact ccaggagggc 20
<210> 35
   <211> 20
   <212> DNA
   <213> artificial
<220>
   <223> oligonucleotide for amplifying aggrecan
<400> 35
   cactgttacc gccacttccc 20
<210> 36
   <211> 20
   <212> DNA
   <213> artificial
<220>
   <223> oligonucleotide for amplifying aggrecan
<400> 36
   accagcggaa gtccccttcg 20

## Description

### Field

The present disclosure relates to methods of treating effects of stroke in a subject.

### Background

Stroke is the second leading cause of mortality after heart disease and the leading cause of disability in Australia. It is the third leading cause of death in the United States, with over 140,000 people dying each year from stroke. It is also the leading cause of serious, long term disability in the United States. Projected costs for stroke in USA for the period from 2005 to 2050 are US$2.2 trillion.

Disability affects 75% of stroke survivors enough to decrease their employability. Stroke can affect subjects physically, mentally, emotionally, or a combination of the three.

Some of the physical disabilities that can result from stroke include muscle weakness, numbness, pressure sores, pneumonia, incontinence, apraxia (inability to perform learned movements), difficulties carrying out daily activities, appetite loss, speech loss, vision loss, and pain. If the stroke is severe enough, or in a certain location such as parts of the brainstem, coma or death can result.

Emotional problems resulting from stroke can result from direct damage to emotional centers in the brain or from frustration and difficulty adapting to new limitations. Post-stroke emotional difficulties include depression, anxiety, panic attacks, flat affect (failure to express emotions), mania, apathy, and psychosis.

Cognitive deficits resulting from stroke include perceptual disorders, speech problems, dementia, and problems with attention and memory. A stroke sufferer may be unaware of his or her own disabilities, a condition called anosognosia. In a condition called hemispatial neglect, a patient is unable to attend to anything on the side of space opposite to the damaged hemisphere.

Up to 10% of all stroke patients develop seizures, most commonly in the week subsequent to the event. The severity of the stroke increases the likelihood of a seizure.

Stroke is the rapidly developing loss of brain function(s) due to disturbance in the blood supply to the brain. This can be due to ischemia (lack of blood flow) caused by blockage (thrombosis, arterial embolism), or a hemorrhage (leakage of blood). As a result, the affected area of the brain is unable to function, which might result in a subject's inability to move one or more limbs on one side of the body, inability to understand or formulate speech, or an inability to see one side of the visual field. Stroke often results in neuronal cell death and can lead to death.

There are two common types of stroke: (i) ischemic stroke, which is caused by a temporary or permanent occlusion to blood flow to the brain, and accounts for 85% of stroke cases, and (ii) hemorrhagic stroke, which is caused by a ruptured blood vessel and accounts for the majority of the remaining cases. The most common cause of ischemic stroke is occlusion of the middle cerebral artery (the intra-cranial artery downstream from the internal carotid artery), which damages cerebrum (e.g., cerebral cortex), e.g., the motor and sensory cortices of the brain. Such damage results in hemiplegia, hemi-anesthesia and, depending on the cerebral hemisphere damaged, either language or visuo-spatial deficits.

Current treatments for ischemic stroke have focused on rescue of the ischemic penumbra, i.e., the moderately hypoperfused region of the brain that retains structural integrity but has lost electrical function. At present, there are only four clinically proven treatments to improve outcomes following stroke:
- aspirin to prevent a further stroke,
- acute clot thrombolysis with tissue plasminogen activator to reverse the occlusion,
- management in a stroke unit, and
- hemicraniectomy to reduce intracranial pressure.

However, such treatments only attempt to mitigate ongoing neurological damage, and do not restore lost neurons or brain function.

Some neuroprotective agents have been tested for efficacy in treatment of stroke, and have failed, including N-methyl-D-aspartate receptor antagonists, nalmefene, lubeluzole, clomethiazole, calcium channel blockers (including a-amino-3-hydroxy-5-rnethylisoxazole-4-proprionic acid antagonists, serotonin agonists (e.g., repinotan), and transmembrane potassium channel modulators), tirilazad, anti-ICAM-I antibody, human antileukocytic antibody (Hu23F2G), antiplatelet antibody (e.g., abciximab), citicoline (an exogenous form of cytidine-5'-diphosphocholine), and basic fibroblast growth factor.

International Patent Application Publication No. WO 2008/036372 A2 discloses methods, cells, and compositions of matter for performing stem cell transplants in patients that have not been previously immunosuppressed.

International Patent Application Publication No. WO 2008/052046 A2 discloses cell delivery compositions comprising a progenitor cell and a targeting moiety, and methods related thereto.

International Patent Application Publication No. WO 2008/148105 A1 discloses pluripotent stem cells and methods for making and using pluripotent stem cells, which pluripotent stem cells, among other things, can differentiate into various cell lineages *in vitro*, *ex vivo* and *in vivo.*

International Patent Application Publication No. WO 2004/084921 A1 discloses the isolation of mesenchymal precursors cells from perivascular niches from a range of tissues utilizing a perivascular marker, such as 3G5, and preferably also alpha smooth muscle actin together with early developmental markers such as STRO-1 and CD146/MUC18.

International Patent Application Publication No. WO 2011/007348 A2 provides a method including obtaining a population of antigen-presenting cells, enriching a population of stem/progenitor cells within a larger population of cells, activating the population of antigen-presenting cells and, following the activating, inducing at least one process selected from the group consisting of: differentiation, expansion, activation, secretion of a molecule, and expression of a marker, by exposing the enriched stem/progenitor cell population to the population of antigen-presenting cells.

Bakondi et al., 2009, Molecular Therapy 17:1938-1947 discloses that CD133⁺ identifies a human bone marrow stem/progenitor cell sub-population with a repertoire of secreted factors that protect against stroke.

It will be apparent from the foregoing that there is a need in the art for therapeutics for stroke. A desirable therapeutic will repair at least some neurological damage caused by stroke and/or restore at least some brain function lost as a result of stroke.

### Summary

The present invention is directed to the subject-matter set forth in the appended claims.

The present disclosure is based on the inventors' identification of cell populations that are useful for the treatment of effects of stroke. As exemplified herein, the inventors have shown that STRO-1⁺ cells and/or progeny thereof and/or factors secreted therefrom are capable of restoring lost brain function following stroke. Exemplary STRO-1⁺ cells are derived from bone marrow and/or dental pulp and can be cultured and/or stored before their use in therapy. The inventors used an accepted animal model for ischemic stroke, e.g., affecting the cerebrum, to demonstrate the efficacy of the cells and/or secreted factors to treat effects of stroke.

For example, the inventors showed that they could restore cerebral function and/or treat a movement disorder following a stroke. The inventors also showed that STRO-1⁺ cells secrete factors (e.g., SDF-1), which cause growth of axons from neurons. Without being bound by any theory or mode of action, the inventors propose that these factors may contribute to neuroprotection, angiogenesis, immune-modulation and/or neuroplasticity.

The inventors also showed that STRO-1⁺ cells are capable of differentiating into neuronal-like cells. However, following injection into the brain of a subject suffering from stroke, few of these cells survive beyond several weeks. These results indicate that STRO-1⁺ cells and/or progeny thereof and/or factors secreted therefrom contribute to survival and/or regeneration of the subject's own neurons following stroke.

The findings presented herein provide the basis for methods for treating one or more effects of stroke. For example, such methods comprise administering to a subject who has suffered a stroke a population of cells enriched for STRO-1⁺ cells and/or progeny thereof and/or soluble factors derived therefrom.

The present disclosure provides method of improving cerebral function or preventing loss of cerebral function in a subject who has suffered a stroke, the method comprising administering to the subject a population of cells enriched for STRO-1⁺ cells and/or progeny thereof and/or soluble factors derived therefrom.

In one example, improving the cerebral function in the subject improves a movement disorder in the subject. In one example, the cerebral function, is cerebral cortex function, such as, motor cortex function and/or sensory cortex function.

The present disclosure additionally or alternatively provides a method of treating or preventing a movement disorder in a subject who has suffered a stroke, the method comprising administering to the subject a population of cells enriched for STRO-1⁺ cells and/or progeny thereof and/or soluble factors derived therefrom.

In one example of a method described herein, the movement disorder is paralysis, partial paralysis, slurred speech, uncoordinated movement, muscle weakness, hypotonicity, hypertonicity or involuntary abnormal movement.

The present disclosure additionally provides a method of regenerating cerebral neurons in a subject who has suffered a stroke, the method comprising administering to the subject a population of cells enriched for STRO-1⁺ cells and/or progeny thereof and/or soluble factors derived therefrom.

The present disclosure additionally provides a method of treating, reducing or preventing cerebral atrophy in a subject who has suffered a stroke, the method comprising administering to the subject a population of cells enriched for STRO-1⁺ cells and/or progeny thereof and/or soluble factors derived therefrom. In one example, the atrophy is in the corpus callosum.

In one example, the cerebral neurons regenerated by performing a method of the disclosure are in the cerebral cortex, for example, in the motor cortex and/or sensory cortex.

In one example, the stroke is an ischemic stroke.

An exemplary stroke contemplated by the present disclosure is an ischemic stroke caused by an occlusion of a middle cerebral artery in a subject.

In one example, a method of the present disclosure comprises administering to the subject the population of cells enriched for STRO-1⁺ cells and/or progeny thereof and/or soluble factors derived therefrom between 1 hour and 1 month, for example, between 1 hour and 2 weeks, such as between 1 hour and 1 week after the stroke. For example, the population of cells enriched for STRO-1⁺ cells and/or progeny thereof and/or soluble factors derived therefrom are administered within about 72 hours after the stroke. For example, the population of cells enriched for STRO-1⁺ cells and/or progeny thereof and/or soluble factors derived therefrom are administered within about 48 hours after the stroke.

In another example, the method comprises administering to the subject the population of cells enriched for STRO-1⁺ cells and/or progeny thereof and/or soluble factors derived therefrom within 24 hours after the stroke. For example, the population of cells enriched for STRO-1⁺ cells and/or progeny thereof and/or soluble factors derived therefrom are administered at about 24 hours after the stroke.

In one example, the method comprises administering a population of cells enriched for STRO-1^{bright} cells and/or progeny thereof and/or soluble factors derived therefrom. In one example, the progeny are additionally enriched for STRO-1^{bright} cells.

Exemplary cells and/or progeny additionally express tissue non-specific alkaline phosphatase (TNAP) and/or heat shock protein 90β (HSP90β) and/or CD146.

In one example, the population of cells is derived from bone marrow or dental pulp.

In one example, the population enriched for STRO-1⁺ cells and/or progeny thereof and/or soluble factors derived therefrom are administered systemically.

In an alternative, example, the population enriched for STRO-1⁺ cells and/or progeny thereof and/or soluble factors derived therefrom are administered locally to the brain of the subject. For example, the population enriched for STRO-1⁺ cells and/or progeny thereof and/or soluble factors derived therefrom are administered locally to the cerebrum of a subject. In one example, the population and/or progeny and/or soluble factors are additionally (or alternatively) administered to the striatum of the subject.

In one example, the population enriched for STRO-1⁺ cells and/or progeny thereof are administered to a site remote from the site of the stroke and migrate to the site of the stroke, e.g., to the ischemic infarct or boundary zone surrounding the ischemic infarct.

An exemplary method described herein according to any example, comprises administering a dose of the population and/or the progeny and/or the soluble factors sufficient to improve cerebral function and/or regenerate cerebral neurons.

In one example, the method comprises administering an effective dose or a therapeutically effective dose of the population and/or progeny and/or soluble factors.

In one example, the method comprises administering between 0.1 x 10⁶ to 5 x 10⁶ STRO-1⁺ cells and/or progeny thereof per kg. For example, the method comprises administering between 0.3 x 10⁶ to 2 x 10⁶ STRO-1⁺ cells and/or progeny thereof per kg. For example, the method comprises administering between 0.5 x 10⁶ to 2 x 10⁶ STRO-1⁺ cells and/or progeny thereof per kg. For example, the method comprises administering between 0.5 x 10⁶ to 1.5 x 10⁶ STRO-1⁺ cells and/or progeny thereof per kg. For example, the method comprises administering about 5 x 10⁵ STRO-1⁺ cells and/or progeny thereof per kg or about 1.5 x 10⁶ STRO-1⁺ cells and/or progeny thereof per kg. For example, the method comprises administering about 1.8 x 10⁶ STRO-1⁺ cells and/or progeny thereof per kg.

In one example, a method described herein according to any example, comprises administering a low dose of STRO-1⁺ cells and/or progeny thereof. For example, the low dose of STRO-1⁺ cells and/or progeny thereof comprises between 0.1 x 10⁵ and 0.5 x 10⁶ STRO-1⁺ cells and/or progeny thereof per kg.

In one example, the population and/or the progeny and/or the soluble factors are administered a plurality of times. For example, the population and/or the progeny and/or the soluble factors are administered once every four or more weeks.

In one example, less than 25% of the STRO-1⁺ cells and/or progeny thereof remain in the brain (e.g., cerebrum) of the subject 28 days following administration. For example, less than 10% of the STRO-1⁺ cells and/or progeny thereof remain in the brain (e.g., cerebrum) of the subject 28 days following administration. For example, less than 5% or 3% of the STRO-1⁺ cells and/or progeny thereof remain in the brain (e.g., cerebrum) of the subject 28 days following administration.

In one example, the population enriched for STRO-1⁺ cells and/or progeny cells are autogeneic or allogeneic and/or the soluble factors can be derived from autogeneic or allogeneic cells. In one example, a method described herein according to any example, comprises isolating or enriching the population and/or progeny and/or isolating the soluble factors.

In examples of a method described herein according to any example, the population enriched for STRO-1⁺ cells and/or progeny cells have been culture expanded prior to administration and/or prior to obtaining the soluble factors. In one example, a method described herein additionally comprises culturing the population and/or progeny. In one example, the method additionally comprises storing the population and/or progeny.

In one example, the population and/or progeny cells thereof and/or soluble factors derived therefrom are administered in the form of a composition comprising said STRO-1⁺ cells and/or progeny cells thereof and/or soluble factors derived therefrom and a carrier and/or excipient. In one example, a method described herein according to any example additionally comprises formulating the population and/or progeny and/or soluble factors with a carrier and/or excipient.

In one example, a method described herein according to any example additionally comprises testing cerebral function of the subject following treatment. For example, the method additionally comprises testing movement and/or strength and/or speech and/or strength of the subject. In one example, if the cerebral function of the subject has not significantly increased compared to before administration of the population and/or progeny and/or soluble factors, the method comprises administering a further dose of the population and/or progeny and/or soluble factors.

In one example, the subject being treated is a mammal, such as a primate, for example, a human.

The present disclosure also provides a population of cells enriched for STRO-1⁺ cells and/or progeny thereof and/or soluble factors derived therefrom for use in improving cerebral function in a subject following a stroke or treating a movement disorder in a subject following a stroke or regenerating cerebral neurons in a subject following a stroke.

The present disclosure additionally provides for a use of a population of cells enriched for STRO-1⁺ cells and/or progeny thereof and/or soluble factors derived therefrom in the manufacture of a medicament for improving cerebral function in a subject following a stroke or treating a movement disorder in a subject following a stroke or regenerating cerebral neurons in a subject following a stroke.

The present disclosure also provides a kit comprising a population of cells enriched for STRO-1⁺ cells and/or progeny thereof and/or soluble factors derived therefrom packaged with instructions for use in a method described herein according to any example.

For example, the present disclosure provides a kit comprising a composition comprising the population and/or the progeny and/or the soluble factors packaged with product information indicating use of the composition in a method described herein according to any example.

The present disclosure also provides a kit for isolating or culturing STRO-1⁺ cells and/or progeny thereof packaged with instructions for use in a method described herein according to any example.

The present disclosure also provides a method comprising providing a population of cells enriched for STRO-1⁺ cells and/or progeny thereof and/or soluble factors derived therefrom with instructions for use in a method described herein according to any example.

The present disclosure also provides a method comprising isolating or offering to isolate or storing or offering to store a population of cells enriched for STRO-1⁺ cells and/or progeny thereof and/or soluble factors derived therefrom for later use in a method described herein according to any example.

The methods described herein are to be taken to apply *mutatis mutandis* to methods for reducing the risk of a further stroke.

### Brief Description of the Drawings

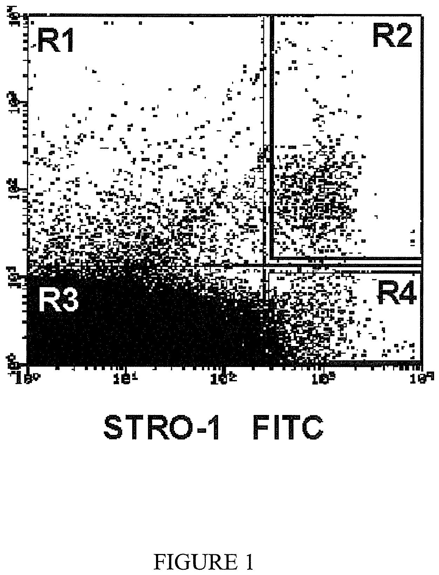

**Figure 1****.** Co-expression of TNAP (STRO-3) and the Mesenchymal Precursor Cell Marker, STRO-1^{bright} by Adult Human bone marrow morphonuclear cells (BMMNC). Dual-color immunofluorescence and flow cytometry was performed by incubation of STRO-1 MACS-selected BMMNC and indirectly labeled with a goat anti-murine IgM antibody coupled to FITC (x axis), and STRO-3 mAb (murine IgG1) indirectly labeled with a goat anti-murine IgG coupled to PE (y axis). The dot plot histogram represents 5 x 10⁴ events collected as listmode data. The vertical and horizontal lines were set to the reactivity levels of <1.0% mean fluorescence obtained with the isotype-matched control antibodies, 1B5 (IgG) and 1A6.12 (IgM) treated under the same conditions. The results demonstrate that a minor population of STRO-1^{bright} cells co-expressed TNAP (upper right quadrant) while the remaining STRO-1⁺ cells failed to react with the STRO-3 mAb.

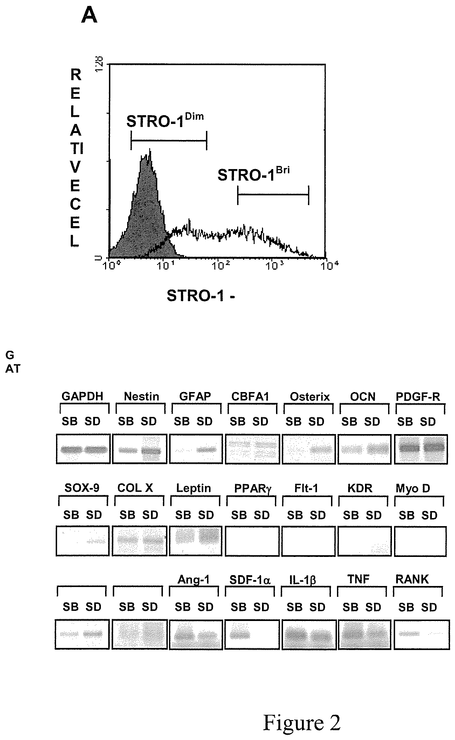

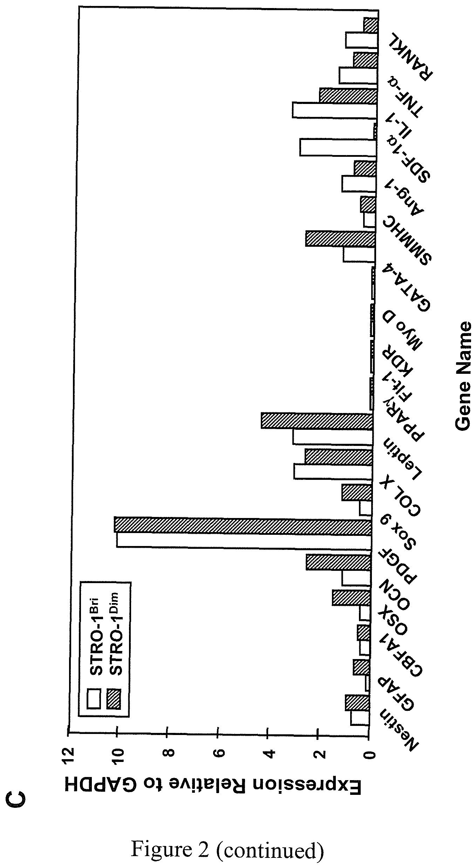

**Figure 2****.** Gene expression profile of STRO-1^{bri} or STRO-1^{dim} progeny of cultured and expanded STRO-1^{bri} MPC. Single cell suspensions of *ex vivo* expanded bone marrow MPC were prepared by trypsin/EDTA treatment. Cells were stained with the STRO-1 antibody which was subsequently revealed by incubation with goat-anti murine IgM-fluorescein isothiocyanate. Total cellular RNA was prepared from purified populations of STRO-1^{dim} or STRO-1^{bri} expressing cells, following fluorescence activated cell sorting (A). Using RNAzolB extraction method, and standard procedures, total RNA was isolated from each subpopulation and used as a template for cDNA synthesis. The expression of various transcripts was assessed by PCR amplification, using a standard protocol as described previously (Gronthos et al. J Cell Sci. 116:1827-1835, 2003). Primers sets used in this study are shown in Table 2. Following amplification, each reaction mixture was analyzed by 1.5% agarose gel electrophoresis, and visualized by ethidium bromide staining (B). Relative gene expression for each cell marker was assessed with reference to the expression of the house-keeping gene, GAPDH, using ImageQant software (C).

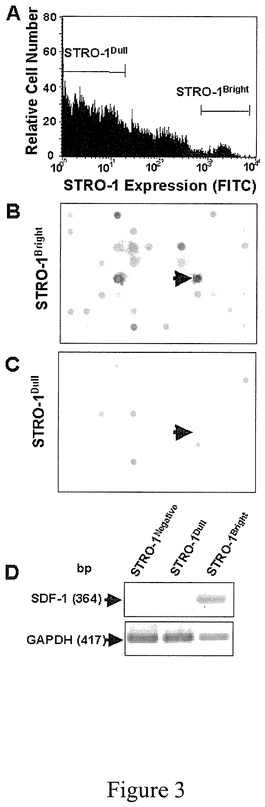

**Figure 3****.** STRO-1^{bri} progeny of cultured and expanded STRO-1⁺MPC express high levels of SDF-1, STRO-1^{dim} progeny do not. (A) MACS-isolated preparations of STRO-1⁺ BMMNCs were partitioned into different STRO-1 subsets according to the regions, STRO-1^{bright} and STRO-1^{dim/dull} using FACS. Total RNA was prepared from each STRO-1 subpopulation and used to construct a STRO-1^{bright} subtraction hybridization library (B-C). Replicate nitrocellulose filters, which have been blotted with representative PCR products amplified from bacterial clones transformed with STRO-1^{bright} subtracted cDNA. The filters were then probed with either [³²P] deoxycytidine triphosphate (dCTP)-labeled STRO-1^{bright} (B) or STRO-1^{dim/dull} (C) subtracted cDNA. The arrows indicate differential expression of 1 clone containing a cDNA fragment corresponding to human SDF-1. (D) Reverse transcriptase (RT)-PCR analysis demonstrating the relative expression of SDF-1 and glyceraldehyde-3-phosphate dehydrogenase (GAPDH) transcripts in total RNA prepared from freshly MACS/FACS-isolated BMMNC STRO-1 populations prior to culture. bp indicates base pair.

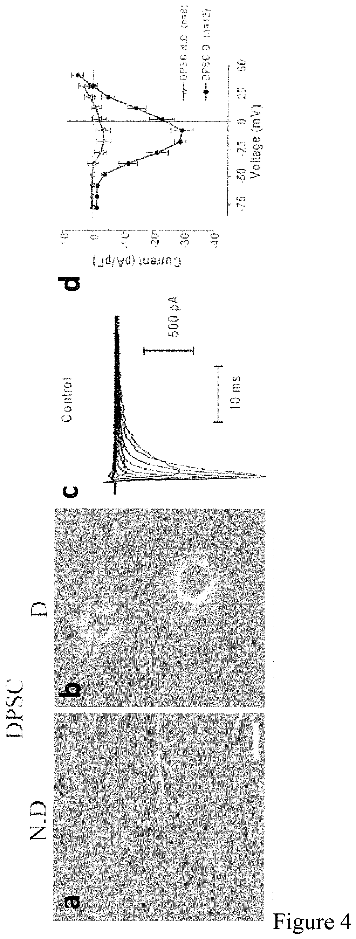

**Figure 4****.** human DPSCs differentiate into neuronal-like cells. (A) Copy of a photomicrograph showing that human foreskin fibroblasts (HFF) do not respond to neuronal-inductive media *in vitro.* (B) Copy of a photomicrograph showing that hDPSC differentiate into a neuronal phenotype in neuronal-inductive media *in vitro.* (C) Graphical representation showing representative examples of traces of Na⁺ currents recorded in differentiated DPSC in response to 30 ms steps ranging from -44 to 36 mV in 10 mV increments. (D) Graphical representation showing average I-V plots of peak Na⁺ currents obtained in differentiated (D) and non-differentiated (N.D.) DPSC in response to voltage steps.

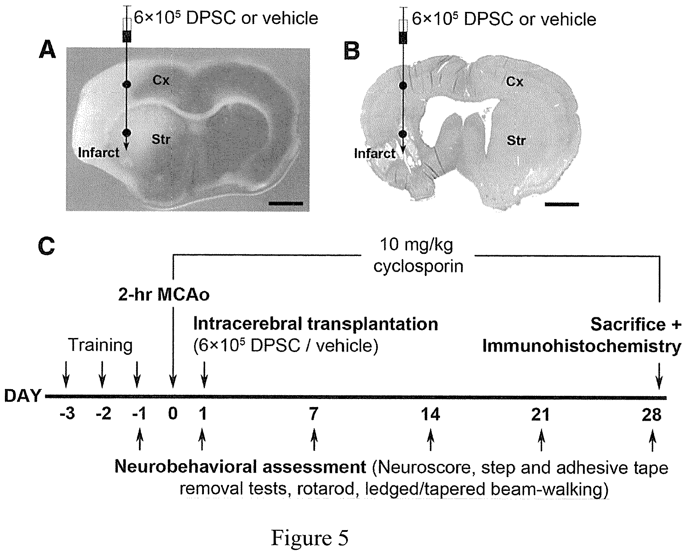

**Figure 5****.** A series of representations depicting the procedure used to treat a model of stroke. Panel A shows a representative coronal rat brain section stained with 2,3,5- triphenyltetrazolium chloride to visualize infarcted (white) tissue 1 day post-middle cerebral artery occlusion (MCAo). Panel B shows a representative coronal rat brain section stained with anti-human mitochondrial antigen antibody and hematoxylin. Black dots in Panels A and B indicate the first striatal injection site at anterior-posterior -0.40 mm, medial-lateral -4.00 mm relative to bregma, dorsal-ventral -5.50mmfrom dura, and the second cortical injection site at dorsal-ventral - 1.75mmfrom dura. Panel C shows a diagrammatic representation of the treatment schedule in which animals were trained for 3 days before MCAo (indicated as day 0), and the third session was used as the preoperative baseline (day -1). Animals were randomly allocated to receive human DPSCs or culture medium alone at 24 hours post-MCAo. Assessment of behavior by investigators blinded to treatment allocation was performed at days 1, 7, 14, 21, and 28 post-MCAo. All animals received a daily subcutaneous injection of 10 mg/kg cyclosporin A throughout the duration of the experiment. Animals were sacrificed, and their brains were processed for immunohistochemistry at the conclusion of neurobehavioral studies. Scale bars=2mm(A, B). Abbreviations: Cx, cerebral cortex; DPSC, dental pulp stem cell; MCAo, middle cerebral artery occlusion; Str, striatum.

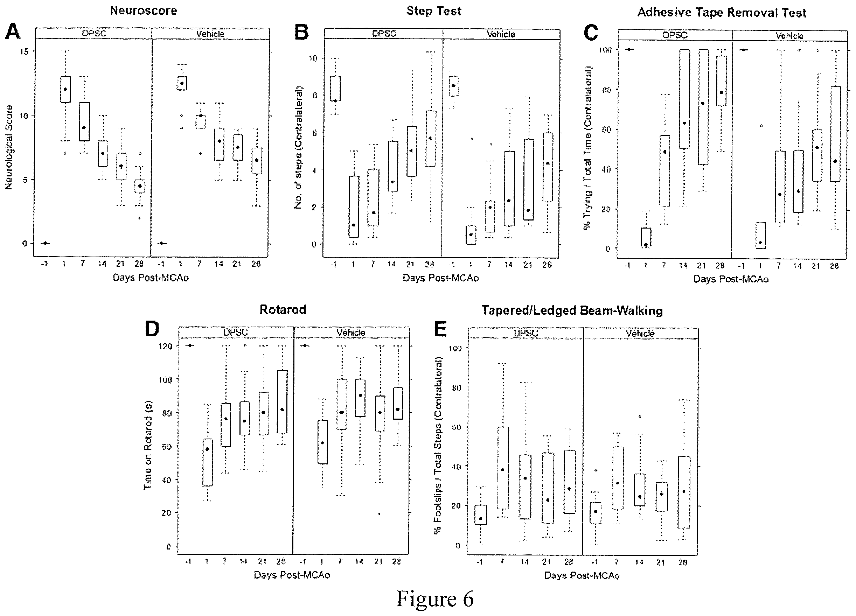

**Figure 6****.** A series of graphical representations showing the effects of human DPSC treatment on post-MCAo neurobehavioral outcome. Following MCAo, all animals exhibited functional deficits in all behavioral tests (day 1 or 7 vs. day -1). Panel A shows at 4 weeks after transplantation, DPSC-treated animals showed significant improvement in gross neurological scores compared with vehicle-treated animals (p < 0.018 groupp x day interaction, repeated-measures analysis of variance [ANOVA]; p<0.05 and p<0.01 between treatment groups at days 21 and 28, respectively; post hoc Fisher's protected least significant difference). As shown in Panel B the DPSC-transplanted group also showed significantly improved forelimb sensorimotor abilities, as demonstrated by a significantly greater number of steps taken with the contralateral forelimb in the step test (p=0.045 overall treatment effect). Panel C also shows that the DPSC-transplanted group also showed a significantly smaller proportion of time attempting to remove the label from the contralateral forepaw in the adhesive tape removal test (p=0.049 overall treatment effect) compared with the vehicle-treated group. Differences in behavioral performance between treatment groups failed to reach statistical significance (p > 0.05, repeated-measures ANOVA) in the rotarod (Panel D) and tapered/ledged beam walking (Panel E) tests. Stroke animals could not complete the beam walking task at day 1 post-MCAo because of circling behavior. Data have been plotted as box plots to visualize the distribution of values. The central asterisk in the box represents the median, and the box represents the middle 50% of values (i.e., it ranges from the 25th to the 75th percentile). The whiskers show the extent of the data, with extreme observations being represented by the outlying circles. Abbreviations: DPSC, dental pulp stem cell; MCAo, middle cerebral artery occlusion.

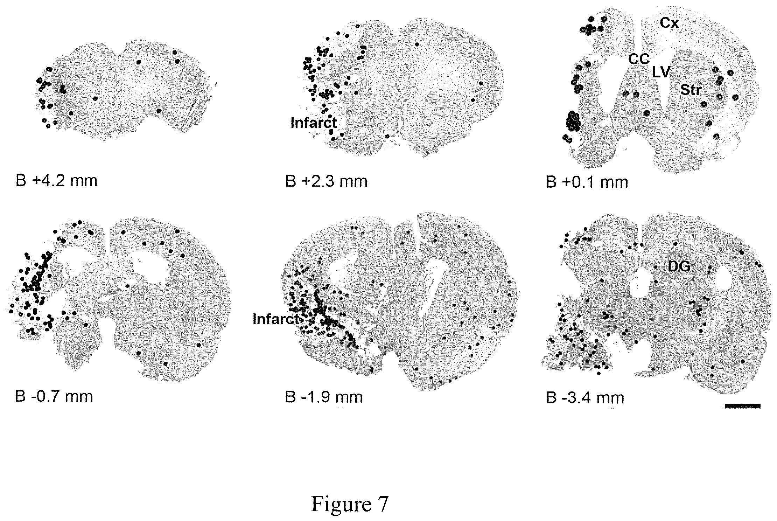

**Figure 7****.** Copies of photographic representations showing distribution of surviving DPSCs in post-ischemic brain. Representative coronal rat brain sections show the survival and migration pattern of transplanted DPSCs (individual cells indicated as black dots) at 4 weeks post-transplantation. Sections were stained for human mitochondrial antigen to detect human cells from rat brain tissue. Note the targeted migration of DPSCs toward the stroke lesion and eventual accumulation around the infarct. Scale bar = 2 mm. Abbreviations: B, bregma; CC, corpus callosum; Cx, cerebral cortex; DG, dentate gyrus of the dorsal hippocampus; LV, lateral ventricle; Str, striatum.

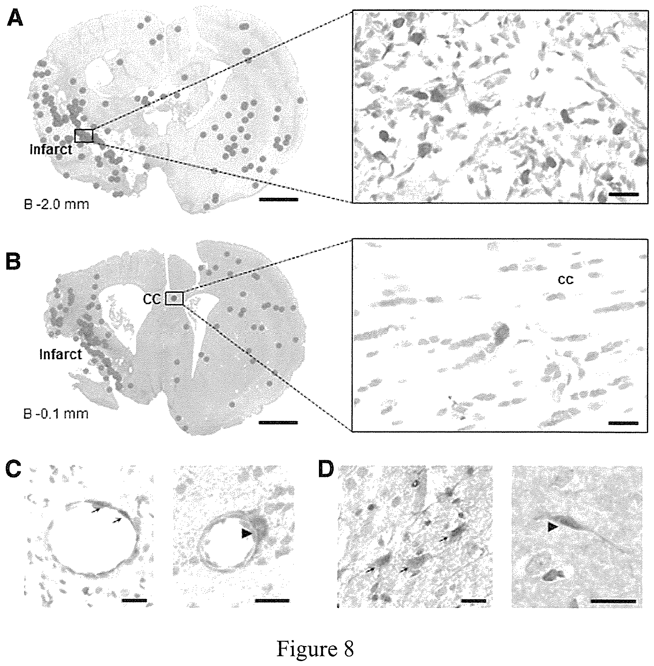

**Figure 8****.** Copies of photographic representations showing the morphology of engrafted human DPSCs at 28 days post-stroke. High magnification of human DPSCs (human mitochondrial antigen-positive cells stained dark grey; right hand side of figure) in the 4-week-post-middle cerebral artery occlusion rodent brain demonstrated accumulation within the immediate vicinity of the infarct core (Panel A) and apparent migration along the CC (Panel B). Note the sparse distribution of DPSCs within the cerebral hemisphere contralateral to the site of injection and stroke. Dots indicate individual human DPSCs. Panel C shows that some DPSC-derived cells appeared to engraft into the wall of cerebral blood vessels, with morphological appearances consistent with endothelial cells (arrows), pericytes, or smooth muscle cells (arrowhead). Panel D shows the morphology of DPSC-derived cells suggested astrocytes (arrows) and a neuron (arrowhead). Scale bars=2mm (Panels A, B) and 25 µm (Panels C, D and =40 high-magnification insets in Panels A, B). Abbreviations: B, bregma; CC, corpus callosum.

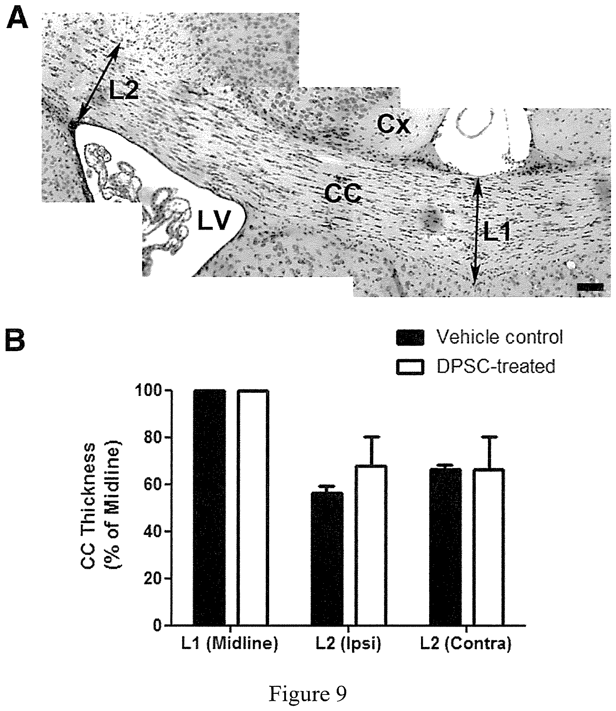

**Figure 9****.** A series of representations showing the effects of DPSC treatment on ipsilesional CC atrophy. Panel A shows a representative rat brain section at the level of bregma in which the two sites used for the measurement of CC thickness at the midline of the brain (L1) and at the lateral edge of the lateral ventricles (L2) in both the ipsilateral and contralateral hemispheres are indicated. Panel B shows that there was a trend toward a decrease of corpus callosal atrophy in the ipsilesional hemisphere of DPSC-treated animals in comparison with vehicle treated animals at 4 weeks post-transplantation. The normalization of L2 (ipsilateral) toward the L1 (midline) CC measurements did not reach statistical significance (p > 0.05). There was no difference in measurements of the contralesional CC thickness between treatment groups. Data are shown as mean±SEM. Scale bar=100 µm. Abbreviations: CC, corpus callosum; Contra, contralateral; Cx, cerebral cortex; DPSC, dental pulp stem cell; Ipsi, ipsilateral; LV, lateral ventricle.

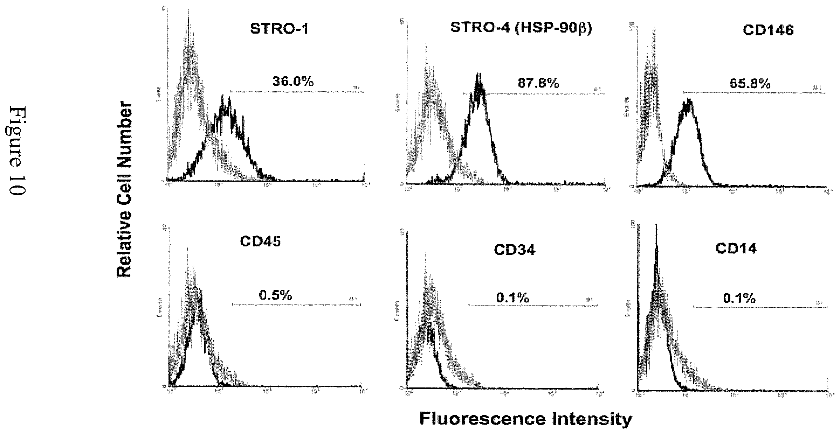

**Figure 10****.** Graphical representations showing representative flow cytometric histograms produced using single cell suspensions of culture expanded bone marrow derived cynomolgus MPCs with positive cell surface expression of the mesenchymal stem cell markers, STRO-1, STRO-4 and CD146 (solid) relative to the isotype (IgM, IgG2a and IgG1) negative controls (hashed) detected using goat anti-murine IgM or IgG conjugated-FITC secondary antibodies. Representative histograms also show that cynomolgus MPCs lack cell surface expression for markers of monocyte/macrophage (CD14), hematopoietic stem/progenitor cells (CD34) and mature leukocyte (CD45).Levels of greater than 1% fluorescence compared to the isotype control signify positivity.

### KEY TO SEQUENCE LISTING

SEQ ID NO: 1 oligonucleotide for amplifying nucleic acid encoding GAPDH
SEQ ID NO: 2 oligonucleotide for amplifying nucleic acid encoding GAPDH
SEQ ID NO: 3 oligonucleotide for amplifying nucleic acid encoding SDF-1
SEQ ID NO: 4 oligonucleotide for amplifying nucleic acid encoding SDF-1
SEQ ID NO: 5 oligonucleotide for amplifying nucleic acid encoding IL-1β
SEQ ID NO: 6 oligonucleotide for amplifying nucleic acid encoding IL-1β
SEQ ID NO: 7 oligonucleotide for amplifying nucleic acid encoding FLT-1
SEQ ID NO: 8 oligonucleotide for amplifying nucleic acid encoding FLT-1
SEQ ID NO: 9 oligonucleotide for amplifying nucleic acid encoding TNF-α
SEQ ID NO: 10 oligonucleotide for amplifying nucleic acid encoding TNF-α
SEQ ID NO: 11 oligonucleotide for amplifying nucleic acid encoding KDR
SEQ ID NO: 12 oligonucleotide for amplifying nucleic acid encoding KDR
SEQ ID NO: 13 oligonucleotide for amplifying nucleic acid encoding RANKL
SEQ ID NO: 14 oligonucleotide for amplifying nucleic acid encoding RANKL
SEQ ID NO: 15 oligonucleotide for amplifying nucleic acid encoding Leptin
SEQ ID NO: 16 oligonucleotide for amplifying nucleic acid encoding Leptin
SEQ ID NO: 17 oligonucleotide for amplifying nucleic acid encoding CBFA-1
SEQ ID NO: 18 oligonucleotide for amplifying nucleic acid encoding CBFA-1
SEQ ID NO: 19 oligonucleotide for amplifying nucleic acid encoding PPARy2
SEQ ID NO: 20 oligonucleotide for amplifying nucleic acid encoding PPARy2
SEQ ID NO: 21 oligonucleotide for amplifying nucleic acid encoding OCN
SEQ ID NO: 22 oligonucleotide for amplifying nucleic acid encoding OCN
SEQ ID NO: 23 oligonucleotide for amplifying nucleic acid encoding MyoD
SEQ ID NO: 24 oligonucleotide for amplifying nucleic acid encoding MyoD
SEQ ID NO: 25 oligonucleotide for amplifying nucleic acid encoding SMMHC
SEQ ID NO: 26 oligonucleotide for amplifying nucleic acid encoding SMMHC
SEQ ID NO: 27 oligonucleotide for amplifying nucleic acid encoding GFAP
SEQ ID NO: 28 oligonucleotide for amplifying nucleic acid encoding GFAP
SEQ ID NO: 29 oligonucleotide for amplifying nucleic acid encoding Nestin
SEQ ID NO: 30 oligonucleotide for amplifying nucleic acid encoding Nestin
SEQ ID NO: 31 oligonucleotide for amplifying nucleic acid encoding SOX9
SEQ ID NO: 32 oligonucleotide for amplifying nucleic acid encoding SOX9
SEQ ID NO: 33 oligonucleotide for amplifying nucleic acid encoding Collagen type X
SEQ ID NO: 34 oligonucleotide for amplifying nucleic acid encoding Collagen type X
SEQ ID NO: 35 oligonucleotide for amplifying nucleic acid encoding Aggrecan
SEQ ID NO: 36 oligonucleotide for amplifying nucleic acid encoding Aggrecan

### Detailed Description

### General Techniques and Selected Definitions

Throughout this specification, unless specifically stated otherwise or the context requires otherwise, reference to a single step, composition of matter, group of steps or group of compositions of matter shall be taken to encompass one and a plurality (i.e. one or more) of those steps, compositions of matter, groups of steps or group of compositions of matter.

Each example of the disclosure is to be applied *mutatis mutandis* to each and every other example unless specifically stated otherwise.

Those skilled in the art will appreciate that the present disclosure is susceptible to variations and modifications other than those specifically described. It is to be understood that the disclosure includes all such variations and modifications. The disclosure also includes all of the steps, features, compositions and compounds referred to or indicated in this specification, individually or collectively, and any and all combinations or any two or more of said steps or features.

The present disclosure is not to be limited in scope by the specific examples described herein, which are intended for the purpose of exemplification only. Functionally-equivalent products, compositions and methods are clearly within the scope of the disclosure.

The present disclosure is performed without undue experimentation using, unless otherwise indicated, conventional techniques of molecular biology, microbiology, virology, recombinant DNA technology, peptide synthesis in solution, solid phase peptide synthesis, and immunology. Such procedures are described, for example, in Sambrook, Fritsch & Maniatis, Molecular Cloning: A Laboratory Manual, Cold Spring Harbor Laboratories, New York, Second Edition (1989), whole of Vols I, II, and III; DNA Cloning: A Practical Approach, Vols. I and II (D. N. Glover, ed., 1985), IRL Press, Oxford, whole of text; Oligonucleotide Synthesis: A Practical Approach (M. J. Gait, ed, 1984) IRL Press, Oxford, whole of text, and particularly the papers therein by Gait, ppl-22; Atkinson *et al*, pp35-81; Sproat *et al*, pp 83-115; and Wu *et al*, pp 135-151; 4. Nucleic Acid Hybridization: A Practical Approach (B. D. Hames & S. J. Higgins, eds., 1985) IRL Press, Oxford, whole of text; Immobilized Cells and Enzymes: A Practical Approach (1986) IRL Press, Oxford, whole of text; Perbal, B., A Practical Guide to Molecular Cloning (1984); Methods In Enzymology (S. Colowick and N. Kaplan, eds., Academic Press, Inc.), whole of series; J.F. Ramalho Ortigao, "The Chemistry of Peptide Synthesis" In: Knowledge database of Access to Virtual Laboratory website (Interactiva, Germany); Sakakibara, D., Teichman, J., Lien, E. Land Fenichel, R.L. (1976). Biochem. Biophys. Res. Commun. 73 336-342; Merrifield, R.B. (1963). J. Am. Chem. Soc. 85, 2149-2154; Barany, G. and Merrifield, R.B. (1979) in The Peptides (Gross, E. and Meienhofer, J. eds.), vol. 2, pp. 1-284, Academic Press, New York. 12. Wünsch, E., ed. (1974) Synthese von Peptiden in Houben-Weyls Metoden der Organischen Chemie (Müler, E., ed.), vol. 15, 4th edn., Parts 1 and 2, Thieme, Stuttgart; Bodanszky, M. (1984) Principles of Peptide Synthesis, Springer-Verlag, Heidelberg; Bodanszky, M. & Bodanszky, A. (1984) The Practice of Peptide Synthesis, Springer-Verlag, Heidelberg; Bodanszky, M. (1985) Int. J. Peptide Protein Res. 25, 449-474; Handbook of Experimental Immunology, VoIs. I-IV (D. M. Weir and C. C. Blackwell, eds., 1986, Blackwell Scientific Publications); and Animal Cell Culture: Practical Approach, Third Edition (John R. W. Masters, ed., 2000), ISBN 0199637970, whole of text.

Throughout this specification, unless the context requires otherwise, the word "comprise", or variations such as "comprises" or "comprising", will be understood to imply the inclusion of a stated step or element or integer or group of steps or elements or integers but not the exclusion of any other step or element or integer or group of elements or integers.

As used herein the term "derived from" shall be taken to indicate that a specified integer may be obtained from a particular source albeit not necessarily directly from that source. In the context of soluble factors derived from STRO-1⁺ cells and/or progeny cells thereof, this term shall be taken to mean one or more factors, e.g., proteins, peptides, carbohydrates, etc, produced during *in vitro* culturing of STRO-1⁺ cells and/or progeny cells thereof.

As used herein, the term "stroke" shall be taken to mean loss of brain function(s), usually rapidly developing, that is due to a disturbance in blood flow to the brain or brainstem. The disturbance can be ischemia (lack of blood) caused by, e.g., thrombosis or embolism, or can be due to a hemorrhage. In one example, the loss of brain function is accompanied by neuronal cell death. In one example, the stroke is caused by a disturbance or loss of blood from to the cerebrum or a region thereof. In one example, a stroke is a neurological deficit of cerebrovascular cause that persists beyond 24 hours or is interrupted by death within 24 hours (as defined by the World Health Organization). Persistence of symptoms beyond 24 hours separates stroke from Transient Ischemic Attack (TIA), in which symptoms persist for less than 24 hours. Symptoms of stroke include hemiplegia (paralysis of one side of the body); hemiparesis (weakness on one side of the body); muscle weakness of the face; numbness; reduction in sensation; altered sense of smell, sense of taste, hearing, or vision; loss of smell, taste, hearing, or vision; drooping of an eyelid (ptosis); detectable weakness of an ocular muscle; decreased gag reflex; decreased ability to swallow; decreased pupil reactivity to light; decreased sensation of the face; decreased balance; nystagmus; altered breathing rate; altered heart rate; weakness in sternocleidomastoid muscle with decreased ability or inability to turn the head to one side; weakness in the tongue; aphasia (inability to speak or understand language); apraxia (altered voluntary movements); a visual field defect; a memory deficit; hemineglect or hemispatial neglect (deficit in attention to the space on the side of the visual field opposite the lesion); disorganized thinking; confusion; development of hypersexual gestures; anosognosia (persistent denial of the existence of a deficit); difficulty walking; altered movement coordination; vertigo; disequilibrium; loss of consciousness; headache; and/or vomiting.

The skilled person will be aware that the "cerebrum" includes the cerebral cortex (or cortices of the cerebral hemispheres), the basal ganglia (or basal nuclei) and limbic system.

The term "cerebral function" includes:
- reasoning, planning, parts of speech, movement, emotions, and problem solving (associated with the frontal lobe);
- movement, orientation, recognition, perception of stimuli (associated with the parietal lobe);
- visual processing (associated with the occipital lobe); and
- perception and recognition of auditory stimuli, memory, and speech (associated with the temporal lobe).

As used herein, the term "movement disorder" shall be taken to mean any change in the ability of a subject to move compared to before a stroke. Exemplary movement disorders include paralysis, partial paralysis, slurred speech, uncoordinated movement, muscle weakness, hypotonicity, hypertonicity or involuntary abnormal movement.

As used herein, "regenerating" neurons will be taken to mean that endogenous neurons of a subject are induced to grow (e.g., to grow axons) and/or divide and/or an endogenous neuronal stem cell is induced to grow and/or divide and/or differentiate to produce new neurons. This is different to an administered cell differentiating to produce a neuron. This term does not mean that a method of regenerating neurons cannot additionally include differentiation of administered cells into neurons or other cell types.

As used herein, the term "effective amount" shall be taken to mean a sufficient quantity of the population enriched for the STRO-1⁺ cells and/or progeny cells thereof and/or soluble factors derived therefrom to induce regeneration of cerebral neurons in a subject who has suffered from a stroke and/or to differentiate into neurons in the subject. An effective amount does not necessarily have to be sufficient to provide a therapeutic benefit on its own, for example, a plurality of administrations of an effective amount of the population and/or cells and/or soluble factors may provide a therapeutic benefit.

As used herein, the term "therapeutically effective amount" shall be taken to mean a sufficient quantity of population enriched for the STRO-1⁺ cells and/or progeny cells thereof and/or soluble factors derived therefrom to improve cerebral function and/or treat a movement disorder and/or regenerate cerebral neurons in a subject who has suffered a stroke.

As used herein, the term "low dose" shall be understood to mean an amount of STRO-1⁺ cells and/or progeny thereof less than 1x10⁶, yet still sufficient to be an "effective amount" as defined herein and/or a "therapeutically effective amount" as defined herein. For example, a low dose comprises 0.5 x 10⁶ or fewer cells, or 0.4 x 10⁶ or fewer cells or 0.3 x10⁶ or fewer cells or 0.1 x 10⁶ or fewer cells.

As used herein, the term "treat" or "treatment" or "treating" shall be understood to mean administering an amount of soluble factors and/or cells and reducing the severity of a movement disorder.

As used herein, the term "prevent" or "preventing" or "prevention" shall be taken to mean administering an amount of soluble factors and/or cells and stopping or hindering or delaying the development or progression of a movement disorder or loss of cerebral function following a stroke. Thus, methods of the present disclosure shall be taken to apply *mutatis mutandis* to methods for preventing a movement disorder or loss of cerebral function in a subject who has suffered a stroke.

As used herein, the term "soluble factors" shall be taken to mean any molecule, e.g., protein, peptide, glycoprotein, glycopeptide, lipoprotein, lipopeptide, carbohydrate, etc. produced by STRO-1⁺ cells and/or progeny thereof that are water soluble. Such soluble factors may be intracellular and/or secreted by a cell. Such soluble factors may be a complex mixture (e.g., supernatant) and/or a fraction thereof and/or may be a purified factor. In one example of the present disclosure soluble factors are or are contained within supernatant. Accordingly, any example herein directed to administration of one or more soluble factors shall be taken to apply *mutatis mutandis* to the administration of supernatant.

As used herein, the term "supernatant" refers to the non-cellular material produced following the *in vitro* culturing of STRO-1⁺ cells and/or progeny thereof in a suitable medium, such as a liquid medium. Typically, the supernatant is produced by culturing the cells in the medium under suitable conditions and time, followed by removing the cellular material by a process such as centrifugation. The supernatant may or may not have been subjected to further purification steps before administration. In one example, the supernatant comprises less than 10⁵, more for example less than 10⁴, such as less than 10³ and for example no live cells.

As used herein, the term "normal or healthy individual" shall be taken to mean a subject who has not suffered a stroke.

In this specification, the term "effect of stroke" will be understood to include and provide literal support for one or more of improving cerebral function, treating or preventing a movement disorder and/or regenerating cerebral neurons.

### STRO-1⁺ Cells or Progeny Cells, and Supernatant or One or More Soluble Factors Derived Therefrom

STRO-1⁺ cells are cells found in bone marrow, blood, dental pulp cells, adipose tissue, skin, spleen, pancreas, brain, kidney, liver, heart, retina, brain, hair follicles, intestine, lung, lymph node, thymus, bone, ligament, tendon, skeletal muscle, dermis, and periosteum; and are capable of differentiating into germ lines such as mesoderm and/or endoderm and/or ectoderm. Exemplary sources of STRO-1⁺ cells are derived from bone marrow and/or dental pulp.

In one example, the STRO-1⁺ cells are multipotential cells which are capable of differentiating into a large number of cell types including, but not limited to, adipose, osseous, cartilaginous, elastic, muscular, and fibrous connective tissues. The specific lineage-commitment and differentiation pathway which these cells enter depends upon various influences from mechanical influences and/or endogenous bioactive factors, such as growth factors, cytokines, and/or local microenvironmental conditions established by host tissues. STRO-1⁺ multipotential cells are thus non-hematopoietic progenitor cells which divide to yield daughter cells that are either stem cells or are precursor cells which in time will irreversibly differentiate to yield a phenotypic cell.

In one example, a STRO-1⁺ cell is capable of differentiating into a neuron or a neuron-like cell.

In one example a STRO-1⁺ cell secretes one or more factors that induce or promote axon growth from a neuron.

In a one example, the STRO-1⁺ cells are enriched from a sample obtained from a subject, e.g., a subject to be treated or a related subject or an unrelated subject (whether of the same species or different). The terms "enriched", "enrichment" or variations thereof are used herein to describe a population of cells in which the proportion of one particular cell type or the proportion of a number of particular cell types is increased when compared with an untreated population of the cells (e.g., cells in their native environment). In one example, a population enriched for STRO-1⁺ cells comprises at least about 0.1% or 0.5% or 1% or 2% or 5% or 10% or 15% or 20% or 25% or 30% or 50% or 75% STRO-1⁺_ cells. In this regard, the term "population of cells enriched for STRO-1⁺ cells" will be taken to provide explicit support for the term "population of cells comprising X% STRO1⁺ cells", wherein X% is a percentage as recited herein. The STRO-1⁺ cells can, in some examples, form clonogenic colonies, e.g. CFU-F (fibroblasts) or a subset thereof (e.g., 50% or 60% or 70% or 70% or 90% or 95%) can have this activity.

In one example, the population of cells is enriched from a cell preparation comprising STRO-1⁺ cells in a selectable form. In this regard, the term "selectable form" will be understood to mean that the cells express a marker (e.g., a cell surface marker) permitting selection of the STRO-1⁺ cells. The marker can be STRO-1, but need not be. For example, as described and/or exemplified herein, cells (e.g., MPCs) expressing STRO-2 and/or STRO-3 (TNAP) and/or STRO-4 and/or VCAM-1 and/or CD146 and/or 3G5 also express STRO-1 (and can be STRO-1^{bright}). Accordingly, an indication that cells are STRO-1⁺ does not mean that the cells are selected by STRO-1 expression. In one example, the cells are selected based on at least STRO-3 expression, e.g., they are STRO-3⁺ (TNAP⁺).

Reference to selection of a cell or population thereof does not require selection from a specific tissue source. As described herein STRO-1⁺ cells can be selected from or isolated from or enriched from a large variety of sources. That said, in some examples, these terms provide support for selection from any tissue comprising STRO-1⁺ cells (e.g., MPCs) or vascularized tissue or tissue comprising pericytes (e.g., STRO-1⁺ pericytes) or any one or more of the tissues recited herein.

In one example, the cells express one or more markers individually or collectively selected from the group consisting of TNAP⁺, VCAM-1⁺, THY-1⁺, STRO-2⁺, STRO-4⁺ (HSP-90β), CD45⁺, CD146⁺, 3G5⁺ or any combination thereof.

In one example, the cells express or the population of cells is enriched for mesenchymal precursor cells expressing STRO-1⁺ (or STRO-1^{bright}) and CD146⁺.

By "individually" is meant that the disclosure encompasses the recited markers or groups of markers separately, and that, notwithstanding that individual markers or groups of markers may not be separately listed herein the accompanying claims may define such marker or groups of markers separately and divisibly from each other.

By "collectively" is meant that the disclosure encompasses any number or combination of the recited markers or groups of peptides, and that, notwithstanding that such numbers or combinations of markers or groups of markers may not be specifically listed herein the accompanying claims may define such combinations or subcombinations separately and divisibly from any other combination of markers or groups of markers.

In one example, the STRO-1⁺ cells are STRO-1^{bright} (*syn.* STRO-1^{bri}). In one example, the Stro-1^{bri} cells are preferentially enriched relative to STRO-1^{dim} or STRO-1^{intermediate} cells.

For example, the STRO-1^{bright} cells are additionally one or more of TNAP⁺, VCAM-1⁺, THY-1⁺, STRO-2⁺, STRO-4⁺ (HSP-90β) and/or CD146⁺. For example, the cells are selected for one or more of the foregoing markers and/or shown to express one or more of the foregoing markers. In this regard, a cell shown to express a marker need not be specifically tested, rather previously enriched or isolated cells can be tested and subsequently used, isolated or enriched cells can be reasonably assumed to also express the same marker.

In one example, the mesenchymal precursor cells are perivascular mesenchymal precursor cells as defined in WO 2004/85630. For example, the mesenchymal precursor cells express a marker of a perivascular cell, e.g., the cells are STRO-1⁺ or STRO-1^{brigh} and/or 3G5⁺. In one example, the cells are or were previously or are progeny of cells that were isolated from vascularized tissue or organs or parts thereof.

A cell that is referred to as being "positive" for a given marker it may express either a low (lo or dim) or a high (bright, bri) level of that marker depending on the degree to which the marker is present on the cell surface, where the terms relate to intensity of fluorescence or other marker used in the sorting process of the cells. The distinction of lo (or dim or dull) and bri will be understood in the context of the marker used on a particular cell population being sorted. A cell that is referred to as being "negative" for a given marker is not necessarily completely absent from that cell. This term means that the marker is expressed at a relatively very low level by that cell, and that it generates a very low signal when detectably labeled or is undetectable above background levels, e.g., levels detected suing an isotype control antibody.

The term "bright", when used herein, refers to a marker on a cell surface that generates a relatively high signal when detectably labeled. Whilst not wishing to be limited by theory, it is proposed that "bright" cells express more of the target marker protein (for example the antigen recognized by STRO-1) than other cells in the sample. For instance, STRO-1^{bri} cells produce a greater fluorescent signal, when labeled with a FITC-conjugated STRO-1 antibody as determined by fluorescence activated cell sorting (FACS) analysis, than non-bright cells (STRO-1^{dull/dim}). For example, "bright" cells constitute at least about 0.1% of the most brightly labeled bone marrow mononuclear cells contained in the starting sample. In other examples, "bright" cells constitute at least about 0.1%, at least about 0.5%, at least about 1%, at least about 1.5%, or at least about 2%, of the most brightly labeled bone marrow mononuclear cells contained in the starting sample. In an example, STRO-1^{bright} cells have 2 log magnitude higher expression of STRO-1 surface expression relative to "background", namely cells that are STRO-1⁻. By comparison, STRO-1^{dim} and/or STRO-1^{intermediate} cells have less than 2 log magnitude higher expression of STRO-1 surface expression, typically about 1 log or less than "background".

As used herein the term "TNAP" is intended to encompass all isoforms of tissue non-specific alkaline phosphatase. For example, the term encompasses the liver isoform (LAP), the bone isoform (BAP) and the kidney isoform (KAP). In one example, the TNAP is BAP. In an example, TNAP as used herein refers to a molecule which can bind the STRO-3 antibody produced by the hybridoma cell line deposited with ATCC on 19 December 2005 under the provisions of the Budapest Treaty under deposit accession number PTA-7282.

Furthermore, in an example of the disclosure, the STRO-1⁺ cells are capable of giving rise to clonogenic CFU-F.

In one example, a significant proportion of the STRO-1⁺ multipotential cells are capable of differentiation into at least two different germ lines. Non-limiting examples of the lineages to which the multipotential cells may be committed include bone precursor cells; hepatocyte progenitors, which are multipotent for bile duct epithelial cells and hepatocytes; neural restricted cells, which can generate glial cell precursors that progress to oligodendrocytes and astrocytes; neuronal precursors that progress to neurons; precursors for cardiac muscle and cardiomyocytes, glucose-responsive insulin secreting pancreatic beta cell lines. Other lineages include, but are not limited to, odontoblasts, dentin-producing cells and chondrocytes, and precursor cells of the following: retinal pigment epithelial cells, fibroblasts, skin cells such as keratinocytes, dendritic cells, hair follicle cells, renal duct epithelial cells, smooth and skeletal muscle cells, testicular progenitors, vascular endothelial cells, tendon, ligament, cartilage, adipocyte, fibroblast, marrow stroma, cardiac muscle, smooth muscle, skeletal muscle, pericyte, vascular, epithelial, glial, neuronal, astrocyte and oligodendrocyte cells.

In another example, the STRO-1⁺ cells are not capable of giving rise, upon culturing, to hematopoietic cells.

In one example, the cells are taken from the subject to be treated, cultured *in vitro* using standard techniques and used to obtain supernatant or soluble factors or expanded cells for administration to the subject as an autologous or allogeneic composition. In an alternative example, cells of one or more of the established human cell lines are used. In another useful example, cells of a non-human animal (or if the patient is not a human, from another species) are used.

The present disclosure also contemplates use of supernatant or soluble factors obtained or derived from STRO-1⁺ cells and/or progeny cells thereof (the latter also being referred to as expanded cells) which are produced from *in vitro* culture. Expanded cells of the disclosure may a have a wide variety of phenotypes depending on the culture conditions (including the number and/or type of stimulatory factors in the culture medium), the number of passages and the like. In certain examples, the progeny cells are obtained after about 2, about 3, about 4, about 5, about 6, about 7, about 8, about 9, or about 10 passages from the parental population. However, the progeny cells may be obtained after any number of passages from the parental population.

The progeny cells may be obtained by culturing in any suitable medium. The term "medium", as used in reference to a cell culture, includes the components of the environment surrounding the cells. Media may be solid, liquid, gaseous or a mixture of phases and materials. Media include liquid growth media as well as liquid media that do not sustain cell growth. Media also include gelatinous media such as agar, agarose, gelatin and collagen matrices. Exemplary gaseous media include the gaseous phase that cells growing on a petri dish or other solid or semisolid support are exposed to. The term "medium" also refers to material that is intended for use in a cell culture, even if it has not yet been contacted with cells. In other words, a nutrient rich liquid prepared for bacterial culture is a medium. A powder mixture that when mixed with water or other liquid becomes suitable for cell culture may be termed a "powdered medium".

In an example, progeny cells useful for the methods of the disclosure are obtained by isolating or enriching TNAP⁺ STRO-1⁺ cells from bone marrow using magnetic beads labeled with the STRO-3 antibody, and then culture expanding the isolated cells (see Gronthos et al. Blood 85: 929-940, 1995 for an example of suitable culturing conditions).

In one example, such expanded cells (progeny) (for example, after at least 5 passages) can be TNAP⁻, CC9⁺, HLA class I⁺, HLA class II⁻, CD14⁻, CD19⁻, CD3⁻, CD11a⁻c⁻, CD31⁻, CD86⁻, CD34⁻ and/or CD80⁻. However, it is possible that under different culturing conditions to those described herein that the expression of different markers may vary. Also, whilst cells of these phenotypes may predominate in the expended cell population it does not mean that there is a minor proportion of the cells do not have this phenotype(s) (for example, a small percentage of the expanded cells may be CC9⁻). In one example, expanded cells still have the capacity to differentiate into different cell types.

In one example, an expended cell population used to obtain supernatant or soluble factors, or cells *per se*, comprises cells wherein at least 25%, such as at least 50%, of the cells are CC9⁺.

In another example, an expanded cell population used to obtain supernatant or soluble factors, or cells *per se*, comprises cells wherein at least 40%, such as at least 45%, of the cells are STRO-1⁺.

In a further example, the expanded cells may express one or more markers collectively or individually selected from the group consisting of LFA-3, THY-1, VCAM-1, ICAM-1, PECAM-1, P-selectin, L-selectin, 3G5, CD49a/CD49b/CD29, CD49c/CD29, CD49d/CD29, CD 90, CD29, CD18, CD61, integrin beta 6-19, thrombomodulin, CD10, CD13, SCF, PDGF-R, EGF-R, IGF1-R, NGF-R, FGF-R, Leptin-R (STRO-2 = Leptin-R), RANKL, STRO-4 (HSP-90β), STRO-1^{bright} and CD146 or any combination of these markers.

In one example, the progeny cells are Multipotential Expanded STRO-1⁺ Multipotential cells Progeny (MEMPs) as defined and/or described in WO 2006/032092. Methods for preparing enriched populations of STRO-1⁺ multipotential cells from which progeny may be derived are described in WO 01/04268 and WO 2004/085630. In an *in vitro* context STRO-1⁺ multipotential cells will rarely be present as an absolutely pure preparation and will generally be present with other cells that are tissue specific committed cells (TSCCs). WO 01/04268 refers to harvesting such cells from bone marrow at purity levels of about 0.1% to 90%. The population comprising MPCs from which progeny are derived may be directly harvested from a tissue source, or alternatively it may be a population that has already been expanded *ex vivo*.

For example, the progeny may be obtained from a harvested, unexpanded, population of substantially purified STRO-1⁺ multipotential cells, comprising at least about 0.1, 1, 5, 10, 20, 30, 40, 50, 60, 70, 80 or 95% of total cells of the population in which they are present. This level may be achieved, for example, by selecting for cells that are positive for at least one marker individually or collectively selected from the group consisting of TNAP, STRO-4 (HSP-90β), STRO-1^{bright}, 3G5⁺, VCAM-1, THY-1, CD146 and STRO-2.

MEMPS can be distinguished from freshly harvested STRO-1⁺ multipotential cells in that they are positive for the marker STRO-1^{bri} and negative for the marker Alkaline phosphatase (ALP). In contrast, freshly isolated STRO-1⁺ multipotential cells are positive for both STRO-1^{bri} and ALP. In one example of the present disclosure, at least 15%, 20%, 30%, 40%, 50%, 60%, 70%, 80%, 90% or 95% of the administered cells have the phenotype STRO-1^{bri}, ALP⁻. In a further example the MEMPS are positive for one or more of the markers Ki67, CD44 and/or CD49c/CD29, VLA-3, α3β1. In yet a further example the MEMPs do not exhibit TERT activity and/or are negative for the marker CD18.

The STRO-1⁺ cell starting population may be derived from any one or more tissue types set out in WO 01/04268 or WO 2004/085630, namely bone marrow, dental pulp cells, adipose tissue and skin, or perhaps more broadly from adipose tissue, teeth, dental pulp, skin, liver, kidney, heart, retina, brain, hair follicles, intestine, lung, spleen, lymph node, thymus, pancreas, bone, ligament, bone marrow, tendon and skeletal muscle.

It will be understood that in performing methods described in the present disclosure, separation of cells carrying any given cell surface marker can be effected by a number of different methods, however, exemplary methods rely upon binding a binding agent (e.g., an antibody or antigen binding fragment thereof) to the marker concerned followed by a separation of those that exhibit binding, being either high level binding, or low level binding or no binding. The most convenient binding agents are antibodies or antibody-based molecules, for example monoclonal antibodies or based on monoclonal antibodies (e.g., proteins comprising antigen binding fragments thereof) because of the specificity of these latter agents. Antibodies can be used for both steps, however other agents might also be used, thus ligands for these markers may also be employed to enrich for cells carrying them, or lacking them.

The antibodies or ligands may be attached to a solid support to allow for a crude separation. In one example, the separation techniques maximize the retention of viability of the fraction to be collected. Various techniques of different efficacy may be employed to obtain relatively crude separations. The particular technique employed will depend upon efficiency of separation, associated cytotoxicity, ease and speed of performance, and necessity for sophisticated equipment and/or technical skill. Procedures for separation may include, but are not limited to, magnetic separation, using antibody-coated magnetic beads, affinity chromatography and "panning" with antibody attached to a solid matrix. Techniques providing accurate separation include but are not limited to FACS. Methods for performing FACS will be apparent to the skilled artisan.

Antibodies against each of the markers described herein are commercially available (e.g., monoclonal antibodies against STRO-1 are commercially available from R&D Systems, USA), available from ATCC or other depositary organization and/or can be produced using art recognized techniques.

In one example, the method for isolating STRO-1⁺ cells comprises a first step being a solid phase sorting step utilizing for example magnetic activated cell sorting (MACS) recognizing high level expression of STRO-1. A second sorting step can then follow, should that be desired, to result in a higher level of precursor cell expression as described in patent specification WO 01/14268. This second sorting step might involve the use of two or more markers.

The method obtaining STRO-1⁺ cells might also include the harvesting of a source of the cells before the first enrichment step using known techniques. Thus the tissue will be surgically removed. Cells comprising the source tissue will then be separated into a so called single cells suspension. This separation may be achieved by physical and or enzymatic means.

Once a suitable STRO-1⁺ cell population has been obtained, it may be cultured or expanded by any suitable means to obtain MEMPs.

In one example, the cells are taken from the subject to be treated, cultured *in vitro* using standard techniques and used to obtain supernatant or soluble factors or expanded cells for administration to the subject as an autologous or allogeneic composition. In an alternative example, cells of one or more of the established human cell lines are used to obtain the supernatant or soluble factors. In another useful example, cells of a non-human animal (or if the patient is not a human, from another species) are used to obtain supernatant or soluble factors.

Methods and uses of the present disclosure can be practiced using cells from any non-human animal species, including but not limited to non-human primate cells, ungulate, canine, feline, lagomorph, rodent, avian, and fish cells. Primate cells with which methods of the disclosure may be performed include but are not limited to cells of chimpanzees, baboons, cynomolgus monkeys, and any other New or Old World monkeys. Ungulate cells with which methods of the disclosure may be performed include but are not limited to cells of bovines, porcines, ovines, caprines, equines, buffalo and bison. Rodent cells with which methods of the disclosure may be performed include but are not limited to mouse, rat, guinea pig, hamster and gerbil cells. Examples of lagomorph species with which methods of the disclosure may be performed include domesticated rabbits, jack rabbits, hares, cottontails, snowshoe rabbits, and pikas. Chickens (*Gallus gallus*) are an example of an avian species with which methods of the disclosure may be performed.

In one example, the cells are human cells.

Cells useful for the methods of the present disclosure may be stored before use, or before obtaining the supernatant or soluble factors. Methods and protocols for preserving and storing of eukaryotic cells, and in particular mammalian cells, are known in the art (cf., for example, Pollard, J. W. and Walker, J. M. (1997) Basic Cell Culture Protocols, Second Edition, Humana Press, Totowa, N.J.; Freshney, R. I. (2000) Culture of Animal Cells, Fourth Edition, Wiley-Liss, Hoboken, N.J.). Any method maintaining the biological activity of the isolated stem cells such as mesenchymal stem/progenitor cells, or progeny thereof, may be utilized in connection with the present disclosure. In one example, the cells are maintained and stored by using cryo-preservation.

### Genetically-Modified Cells

In one example, the STRO-1⁺ cells and/or progeny cells thereof are genetically modified, e.g., to express and/or secrete a protein of interest. For example, the cells are engineered to express a protein useful in the treatment of movement disorders or other effects of stroke, such as, nerve growth factor or peptides described, for example, in Meade et al., J Exp Stroke Transl Med 2: 22-40, 2009.

Methods for genetically modifying a cell will be apparent to the skilled artisan. For example, a nucleic acid that is to be expressed in a cell is operably-linked to a promoter for inducing expression in the cell. For example, the nucleic acid is linked to a promoter operable in a variety of cells of a subject, such as, for example, a viral promoter, e.g., a CMV promoter (e.g., a CMV-IE promoter) or a SV-40 promoter. Additional suitable promoters are known in the art and shall be taken to apply *mutatis mutandis* to the present example of the disclosure.

In one example, the nucleic acid is provided in the form of an expression construct. As used herein, the term "expression construct" refers to a nucleic acid that has the ability to confer expression on a nucleic acid (e.g. a reporter gene and/or a counter-selectable reporter gene) to which it is operably connected, in a cell. Within the context of the present disclosure, it is to be understood that an expression construct may comprise or be a plasmid, bacteriophage, phagemid, cosmid, virus sub-genomic or genomic fragment, or other nucleic acid capable of maintaining and/or replicating heterologous DNA in an expressible format.

Methods for the construction of a suitable expression construct for performance of the disclosure will be apparent to the skilled artisan and are described, for example, in Ausubel et al (In: Current Protocols in Molecular Biology. Wiley Interscience, ISBN 047 150338, 1987) or Sambrook et al (In: Molecular Cloning: Molecular Cloning: A Laboratory Manual, Cold Spring Harbor Laboratories, New York, Third Edition 2001). For example, each of the components of the expression construct is amplified from a suitable template nucleic acid using, for example, PCR and subsequently cloned into a suitable expression construct, such as for example, a plasmid or a phagemid.

Vectors suitable for such an expression construct are known in the art and/or described herein. For example, an expression vector suitable for use in a method of the present disclosure in a mammalian cell is, for example, a vector of the pcDNA vector suite supplied by Invitrogen, a vector of the pCI vector suite (Promega), a vector of the pCMV vector suite (Clontech), a pM vector (Clontech), a pSI vector (Promega), a VP 16 vector (Clontech) or a vector of the pcDNA vector suite (Invitrogen).

The skilled artisan will be aware of additional vectors and sources of such vectors, such as, for example, Life Technologies Corporation, Clontech or Promega.

Means for introducing the isolated nucleic acid molecule or a gene construct comprising same into a cell for expression are known to those skilled in the art. The technique used for a given organism depends on the known successful techniques. Means for introducing recombinant DNA into cells include microinjection, transfection mediated by DEAE-dextran, transfection mediated by liposomes such as by using lipofectamine (Gibco, MD, USA) and/or cellfectin (Gibco, MD, USA), PEG-mediated DNA uptake, electroporation and microparticle bombardment such as by using DNA-coated tungsten or gold particles (Agracetus Inc., WI, USA) amongst others.

Alternatively, an expression construct of the disclosure is a viral vector. Suitable viral vectors are known in the art and commercially available. Conventional viral-based systems for the delivery of a nucleic acid and integration of that nucleic acid into a host cell genome include, for example, a retroviral vector, a lentiviral vector or an adeno-associated viral vector. Alternatively, an adenoviral vector is useful for introducing a nucleic acid that remains episomal into a host cell. Viral vectors are an efficient and versatile method of gene transfer in target cells and tissues. Additionally, high transduction efficiencies have been observed in many different cell types and target tissues.

For example, a retroviral vector generally comprises cis-acting long terminal repeats (LTRs) with packaging capacity for up to 6-10 kb of foreign sequence. The minimum cis-acting LTRs are sufficient for replication and packaging of a vector, which is then used to integrate the expression construct into the target cell to provide long term expression. Widely used retroviral vectors include those based upon murine leukemia virus (MuLV), gibbon ape leukemia virus (GaLV), simian immunodeficiency virus (SrV), human immunodeficiency virus (HIV), and combinations thereof (see, e.g., Buchscher et al., J Virol. 56:2731-2739 (1992); Johann et al, J. Virol 65:1635-1640 (1992); Sommerfelt et al, Virol. 76:58-59 (1990); Wilson et al, J. Virol. 63:274-2318 (1989); Miller et al., J. Virol. 65:2220-2224 (1991); PCT/US94/05700; Miller and Rosman BioTechniques 7:980-990, 1989; Miller, A. D. Human Gene Therapy 7:5-14, 1990; Scarpa et al Virology 75:849-852, 1991; Burns et al. Proc. Natl. Acad. Sci USA 90:8033-8037, 1993).

Various adeno-associated virus (AAV) vector systems have also been developed for nucleic acid delivery. AAV vectors can be readily constructed using techniques known in the art. See, e.g., U.S. Pat. Nos. 5,173,414 and 5,139,941; International Publication Nos. WO 92/01070 and WO 93/03769; Lebkowski et al. Molec. Cell. Biol. 5:3988-3996, 1988; Vincent et al. (1990) Vaccines 90 (Cold Spring Harbor Laboratory Press);Carter Current Opinion in Biotechnology 5:533-539, 1992; Muzyczka. Current Topics in Microbiol, and Immunol. 158:97-129, 1992; Kotin, Human Gene Therapy 5:793-801, 1994; Shelling and Smith Gene Therapy 7:165-169, 1994; and Zhou et al. J Exp. Med. 779:1867-1875, 1994.

Additional viral vectors useful for delivering an expression construct of the disclosure include, for example, those derived from the pox family of viruses, such as vaccinia virus and avian poxvirus or an alphavirus or a conjugate virus vector (e.g. that described in Fisher-Hoch et al., Proc. Natl Acad. Sci. USA 56:317-321, 1989).

### Assaying Therapeutic/Prophylactic Potential of Cells and Soluble Factors

Methods for determining the ability of cells or soluble factors to improve cerebral function and/or treat a movement disorder and/or regenerate cerebral neurons will be apparent to the skilled artisan.

For example, cells or soluble factors are cultured with neurons and the ability of the cells or soluble factors to induce axonal growth is assessed, e.g., microscopic techniques. A similar assay can be performed *in vivo* by injecting the cells and/or soluble factors into the brain of an embryonic chick and assessing growth of axons, e.g., as exemplified herein.

In one example, the neurons are exposed to oxygen and/or glucose deprivation prior to or at the time of culturing with the cells and/or soluble factors. Oxygen and glucose deprivation is a model of ischemia.

In another example, cells are grown under conditions that induce neuronal differentiation, e.g., comprising butylated hydroxanisole and valproic acid or 5-azacytidine or indomethacine and isobutylmethylxanthine and insulin. Cells that differentiate into neurons are considered suitable for therapy.

In a further example, cells or soluble factors (e.g., a mixture of factors or a single factor or a fraction of factors (e.g., derived by affinity purification or chromatography)) are administered to a model of stroke the effect on cerebral function, cerebral neuron regeneration and/or a movement disorder is assessed.

There are various known techniques for inducing an ischemic stroke in a non-human animal subject, such as, aorta/vena cava occlusion, external neck torniquet or cuff, hemorrhage or hypotension, intracranial hypertension or common carotid artery occlusion, two-vessel occlusion and hypotension, four-vessel occlusion, unilateral common carotid artery occlusion (in some species only), endothelin-1-induced constriction of arteries and veins, middle cerebral artery occlusion, spontaneous brain infarction (in spontaneously hypertensive rats), macrosphere embolization, blood clot embolization or microsphere embolization. Hemorrhagic stroke can be modeled by infusion of collagenase into the brain.

In one example, the model of stroke comprises middle cerebral artery occlusion to produce an ischemic stroke.

To test the ability of a population and/or progeny and/or secreted factors to treat the effects of stroke, the population and/or progeny and/or secreted factors is(are) administered following induction of stroke, e.g., within 1 hour to 1 day of stroke. Following administration an assessment of cerebral function and/or movement disorder is made, e.g., on several occasions.

Methods of assessing cerebral function and/or movement disorders will be apparent to the skilled artisan and include, for example, rotarod, elevated plus maze, open-field, Morris water maze, T-maze, the radial arm maze, assessing movement (e.g., area covered in a period of time), tail flick or De Ryck's behavioral test (De Ryck et al., Stroke. 20:1383-1390, 1989). Additional tests will be apparent to the skilled artisan and/or described herein.

In another example, the effect of the population and/or progeny and/or secreted factors on the cerebrum is assessed by imaging. For example, apoptosis, autophagy and neurovascular unit can be detected by *in vivo* optical imaging (Liu et al., Brain Res. 2011 Apr 27), and PET, MRI or CT can be used to assess various factors, such as size of infarct, size of penumbra and/or extent of brain damage.

The effect of the population and/or progeny and/or secreted factors on cerebral neurons is also assessed. For example, the brain or a region of the cerebrum thereof is removed and the number of neurons or a sub-type of neurons is assessed or estimated. Depending on the model used, this number may be compared to the number in a control animal, or if only focal ischemia is induced, to a control region of the brain from the same animal.

### Cellular Compositions

In one example of the present disclosure STRO-1⁺ cells and/or progeny cells thereof are administered in the form of a composition. In one example, such a composition comprises a pharmaceutically acceptable carrier and/or excipient.

The terms "carrier" and "excipient" refer to compositions of matter that are conventionally used in the art to facilitate the storage, administration, and/or the biological activity of an active compound (see, e.g., Remington's Pharmaceutical Sciences, 16th Ed., Mac Publishing Company (1980). A carrier may also reduce any undesirable side effects of the active compound. A suitable carrier is, for example, stable, e.g., incapable of reacting with other ingredients in the carrier. In one example, the carrier does not produce significant local or systemic adverse effect in recipients at the dosages and concentrations employed for treatment.

Suitable carriers for the present disclosure include those conventionally used, e.g., water, saline, aqueous dextrose, lactose, Ringer's solution, a buffered solution, hyaluronan and glycols are exemplary liquid carriers, particularly (when isotonic) for solutions. Suitable pharmaceutical carriers and excipients include starch, cellulose, glucose, lactose, sucrose, gelatin, malt, rice, flour, chalk, silica gel, magnesium stearate, sodium stearate, glycerol monostearate, sodium chloride, glycerol, propylene glycol, water, ethanol, and the like.

In another example, a carrier is a media composition, e.g., in which a cell is grown or suspended. In an example, such a media composition does not induce any adverse effects in a subject to whom it is administered.

Exemplary carriers and excipients do not adversely affect the viability of a cell and/or the ability of a cell to reduce, prevent or delay an effect of stroke.

In one example, the carrier or excipient provides a buffering activity to maintain the cells and/or soluble factors at a suitable pH to thereby exert a biological activity, e.g., the carrier or excipient is phosphate buffered saline (PBS). PBS represents an attractive carrier or excipient because it interacts with cells and factors minimally and permits rapid release of the cells and factors, in such a case, the composition of the disclosure may be produced as a liquid for direct application to the blood stream or into a tissue or a region surrounding or adjacent to a tissue, e.g., by injection.

STRO-1⁺ cells and/or progeny cells thereof can also be incorporated or embedded within scaffolds that are recipient-compatible and which degrade into products that are not harmful to the recipient. These scaffolds provide support and protection for cells that are to be transplanted into the recipient subjects. Natural and/or synthetic biodegradable scaffolds are examples of such scaffolds.

A variety of different scaffolds may be used successfully in the practice of the disclosure. Exemplary scaffolds include, but are not limited to biological, degradable scaffolds. Natural biodegradable scaffolds include collagen, fibronectin, and laminin scaffolds. Suitable synthetic material for a cell transplantation scaffold should be able to support extensive cell growth and cell function. Such scaffolds may also be resorbable. Suitable scaffolds include polyglycolic acid scaffolds, e.g., as described by Vacanti, et al. J. Ped. Surg. 23:3-9 1988; Cima, et al. Biotechnol. Bioeng. 38:145 1991; Vacanti, et al. Plast. Reconstr. Surg. 88:753-9 1991; or synthetic polymers such as polyanhydrides, polyorthoesters, and polylactic acid.

In another example, the cells may be administered in a gel scaffold (such as Gelfoam from Upjohn Company.

The cellular compositions useful for methods described herein may be administered alone or as admixtures with other cells. Cells that may be administered in conjunction with the compositions of the present disclosure include, but are not limited to, other multipotent or pluripotent cells or stem cells, or bone marrow cells. The cells of different types may be admixed with a composition of the disclosure immediately or shortly prior to administration, or they may be co-cultured together for a period of time prior to administration.

In one example, the composition comprises an effective amount or a therapeutically or prophylactically effective amount of cells. For example, the composition comprises about 1x10⁵ STRO-1⁺ cells/kg to about 1x10⁷ STRO-1⁺ cells/kg or about 1x10⁶ STRO-1⁺ cells/kg to about 5x10⁶ STRO-1⁺ cells/kg. The exact amount of cells to be administered is dependent upon a variety of factors, including the age, weight, and sex of the patient, and the extent and severity of the stroke and/or site of the stroke.

In one example, a low dose of cells is administered to the subject. Exemplary dosages include between about 0.1 x 10⁴ and 0.5 x 10⁶ cells per kg, for example, between about 0.1 x 10⁵ and 0.5 x 10⁶ cells per kg, such as, between about 0.5 x 10⁵ and 0.5 x 10⁶ cells per kg, for example, between about 0.1 x 10⁶ and 0.5 x 10⁶ cells per kg, e.g., about 0.2 x 10⁶ or 0.3 x 10⁶ or 0.4 x 10⁶ cells per kg.

In some examples, cells are contained within a chamber that does not permit the cells to exit into a subject's circulation, however that permits factors secreted by the cells to enter the circulation. In this manner soluble factors may be administered to a subject by permitting the cells to secrete the factors into the subject's circulation. Such a chamber may equally be implanted at a site in a subject to increase local levels of the soluble factors, e.g., implanted in or near a pancreas.

In some examples of the disclosure, it may not be necessary or desirable to immunosuppress a patient prior to initiation of therapy with cellular compositions. Accordingly, transplantation with allogeneic, or even xenogeneic, STRO-1⁺ cells or progeny thereof may be tolerated in some instances.

However, in other instances it may be desirable or appropriate to pharmacologically immunosuppress a patient prior to initiating cell therapy and/or reduce an immune response of a subject against the cellular composition. This may be accomplished through the use of systemic or local immunosuppressive agents, or it may be accomplished by delivering the cells in an encapsulated device. The cells may be encapsulated in a capsule that is permeable to nutrients and oxygen required by the cell and therapeutic factors the cell is yet impermeable to immune humoral factors and cells. In one example, the encapsulant is hypoallergenic, is easily and stably situated in a target tissue, and provides added protection to the implanted structure. These and other means for reducing or eliminating an immune response to the transplanted cells are known in the art. As an alternative, the cells may be genetically modified to reduce their immunogenicity.

### Compositions of Soluble Factors

In one example of the present disclosure, STRO-1⁺ cell-derived and/or progeny cell-derived supernatant or soluble factors are administered in the form of a composition, e.g., comprising a suitable carrier and/or excipient. In one example, the carrier or excipient does not adversely affect the biological effect of the soluble factors or supernatant.

In one example, the composition comprises a composition of matter to stabilize a soluble factor or a component of supernatant, e.g., a protease inhibitor. In one example, the protease inhibitor is not included in an amount sufficient to have an adverse effect on a subject.

Compositions comprising STRO-1⁺ cell-derived and/or progeny cell-derived supernatant or soluble factors may be prepared as appropriate liquid suspensions, e.g., in culture medium or in a stable carrier or a buffer solution, e.g., phosphate buffered saline. Suitable carriers are described herein above. In another example, suspensions comprising STRO-1cell-derived and/or progeny cell-derived supernatant or soluble factors are oily suspensions for injection. Suitable lipophilic solvents or vehicles include fatty oils such as sesame oil; or synthetic fatty acid esters, such as ethyl oleate or triglycerides; or liposomes. Suspensions to be used for injection may also contain substances which increase the viscosity of the suspension, such as sodium carboxymethyl cellulose, sorbitol, or dextran. Optionally, the suspension may also contain suitable stabilizers or agents which increase the solubility of the compounds to allow for the preparation of highly concentrated solutions.

Sterile injectable solutions can be prepared by incorporating the supernatant or soluble factors in the required amount in an appropriate solvent with one or a combination of ingredients described above, as required, followed by filtered sterilization.

Generally, dispersions are prepared by incorporating the supernatant or soluble factors into a sterile vehicle that contains a basic dispersion medium and the required other ingredients from those enumerated above. In the case of sterile powders for the preparation of sterile injectable solutions, exemplary methods of preparation are vacuum drying and freeze-drying which yields a powder of the active ingredient plus any additional desired ingredient from a previously sterile-filtered solution thereof. In accordance with an alternative aspect of the disclosure, the supernatant or soluble factors may be formulated with one or more additional compounds that enhance its solubility.

Other exemplary carriers or excipients are described, for example, in Hardman, et al. (2001) Goodman and Gilman's The Pharmacological Basis of Therapeutics, McGraw-Hill, New York, N. Y.; Gennaro (2000) Remington: The Science and Practice of Pharmacy, Lippincott, Williams, and Wilkins, New York, N. Y.; Avis, et al. (eds.) (1993) Pharmaceutical Dosage Forms: Parenteral Medications, Marcel Dekker, NY; Lieberman, et al. (eds.) (1990) Pharmaceutical Dosage Forms: Tablets, Marcel Dekker, NY; Lieberman, et al. (eds.) (1990) Pharmaceutical Dosage Forms: Disperse Systems, Marcel Dekker, NY; Weiner and Kotkoskie (2000) Excipient Toxicity and Safety, Marcel Dekker, Inc., New York, N. Y.

Therapeutic compositions typically should be sterile and stable under the conditions of manufacture and storage. The composition can be formulated as a solution, microemulsion, liposome, or other ordered structure. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. In many cases, it will be desirable to include isotonic agents, for example, sugars, polyalcohols such as mannitol, sorbitol, or sodium chloride in the composition. Prolonged absorption of the injectable compositions can be brought about by including in the composition an agent which delays absorption, for example, monostearate salts and gelatin. Moreover, the soluble factors may be administered in a time release formulation, for example in a composition which includes a slow release polymer. The active compounds can be prepared with carriers that will protect the compound against rapid release, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, polylactic acid and polylactic, polyglycolic copolymers (PLG). Many methods for the preparation of such formulations are patented or generally known to those skilled in the art.

The supernatant or soluble factors may be administered in combination with an appropriate matrix, for instance, to provide slow release of the soluble factors.

### Additional Components of Compositions

The STRO-1⁺ cell-derived supernatant or soluble factors, STRO-1⁺ cells or progeny thereof may be administered with other beneficial drugs or biological molecules (growth factors, trophic factors). When administered with other agents, they may be administered together in a single pharmaceutical composition, or in separate pharmaceutical compositions, simultaneously or sequentially with the other agents (either before or after administration of the other agents). Bioactive factors which may be co-administered include anti-apoptotic agents (e.g., EPO, EPO mimetibody, TPO, IGF-I and IGF-II, HGF, caspase inhibitors); anti-inflammatory agents (e.g., p38 MAPK inhibitors, TGF-beta inhibitors, statins, IL-6 and IL-1 inhibitors, PEMIROLAST, TRANILAST, REMICADE, SIROLIMUS, and NSAIDs (non-steroidal anti-inflammatory drugs; e.g., TEPOXALIN, TOLMETIN, SUPROFEN); immunosupressive/immunomodulatory agents (e.g., calcineurin inhibitors, such as cyclosporine, tacrolimus; mTOR inhibitors (e.g., SIROLIMUS, EVEROLIMUS); anti-proliferatives (e.g., azathioprine, mycophenolate mofetil); corticosteroids (e.g., prednisolone, hydrocortisone); antibodies such as monoclonal anti-IL-2Ralpha receptor antibodies (e.g., basiliximab, daclizumab), polyclonal anti-T-cell antibodies (e.g., anti-thymocyte globulin (ATG); anti-lymphocyte globulin (ALG); monoclonal anti-T cell antibody OKT3)); anti-thrombogenic agents (e.g., heparin, heparin derivatives, urokinase, PPack (dextrophenylalanine proline arginine chloromethylketone), antithrombin compounds, platelet receptor antagonists, anti-thrombin antibodies, antiplatelet receptor antibodies, aspirin, dipyridamole, protamine, hirudin, prostaglandin inhibitors, and platelet inhibitors); and anti-oxidants (e.g., probucol, vitamin A, ascorbic acid, tocopherol, coenzyme Q-10, glutathione, L-cysteine, N-acetylcysteine) as well as local anesthetics.

In one example, a composition as described herein according to any example comprises an additional factor for improving cerebral function and/or regenerating cerebral neurons and/or treating a movement disorder, e.g., a nerve growth factor.

Alternatively, or in addition, cells, secreted factors and/or a composition as described herein according to any example is combined with a known treatment of stroke effects, e.g., physiotherapy and/or speech therapy.

In one example, a pharmaceutical composition as described herein according to any example comprises a compound used to treat effects of a stroke. Alternatively, a method of treatment/prophylaxis as described herein according to any example of the disclosure additionally comprises administering a compound used to treat effects of a stroke. Exemplary compounds are described herein and are to be taken to apply *mutatis mutandis* to these examples of the present disclosure.

In another example, a composition as described herein according to any example additionally comprises a factor that induces or enhances differentiation of a progenitor cell into a vascular cell. Exemplary factors include, vascular endothelial growth factor (VEGF), platelet derived growth factor (PDGF; e.g., PDGF-BB), and FGF.

In another example, a composition as described herein according to any example additionally comprises a tissue specific committed cell (TSCC). In this respect, International Patent Application No. PCT/AU2005/001445 demonstrates that administration of a TSCC and a STRO-1⁺ cells can lead to enhanced proliferation of the TSCC. In one example, the TSCC is a vascular cell. Administration of such a composition to a subject may lead to increased production of vasculature, e.g., leading to increased nutrients being delivered to the affected tissue.

### Medical Devices

The present disclosure also provides medical devices for use or when used in a method as described herein according to any example. For example, the present disclosure provides a syringe or catheter or other suitable delivery device comprising STRO-1⁺ cells and/or progeny cells thereof and/or soluble factors therefrom and/or a composition as described herein according to any example. Optionally, the syringe or catheter is packaged with instructions for use in a method as described herein according to any example.

In another example, the present disclosure provides an implant comprising STRO-1⁺ cells and/or progeny cells thereof and/or soluble factors therefrom and/or a composition as described herein according to any example. Optionally, the implant is packaged with instructions for use in a method as described herein according to any example. Suitable implants may be formed with a scaffold, e.g., as described herein above and STRO-1⁺ cells and/or progeny cells thereof and/or soluble factors therefrom.

### Modes of Administration

The STRO-1⁺ cell-derived supernatant or soluble factors, STRO-1⁺ cells or progeny thereof may be surgically implanted, injected, delivered (e.g., by way of a catheter or syringe), or otherwise administered directly or indirectly to the site in need of repair or augmentation, e.g., into a fat pad.

In on example, the STRO-1⁺ cell-derived supernatant or soluble factors, STRO-1⁺ cells or progeny thereof is/are delivered to the blood stream of a subject. For example, the STRO-1⁺ cell-derived supernatant or soluble factors, STRO-1⁺ cells or progeny thereof are delivered parenterally. Exemplary routes of parenteral administration include, but are not limited to, intraperitoneal, intraventricular, intracerebroventricular, intrathecal, or intravenous. In one example, the STRO-1⁺ cell-derived supernatant or soluble factors, STRO-1⁺ cells or progeny thereof are delivered intra-arterially, into an aorta, into an atrium or ventricle of the heart or into a blood vessel, e.g., intravenously.

In the case of cell delivery to an atrium or ventricle of the heart, cells can be administered to the left atrium or ventricle to avoid complications that may arise from rapid delivery of cells to the lungs.

In one example, the population and/or progeny and/or soluble factors are administered into the carotid artery.

In one example, the population and/or progeny and/or soluble factors are administered into the brain of a subject, e.g., intra-cranially. For example, the population and/or cells and/or soluble factors are administered into the cerebrum and/or optionally into the striatum.

In one example, the population and/or cells and/or soluble factors are administered into the site of ischemia and/or peripheral to the site of ischemia. Such sites can be determined using methods known in the art, e.g., magnetic resonance imaging and/or computed tomography scanning and/or ultrasound.

Selecting an administration regimen for a therapeutic formulation depends on several factors, including the serum or tissue turnover rate of the entity, the level of symptoms, and the immunogenicity of the entity.

In one example, STRO-1⁺ cell-derived supernatant or soluble factors, STRO-1⁺ cells or progeny thereof are delivered as a single bolus dose. Alternatively, STRO-1⁺ cell-derived supernatant or soluble factors, STRO-1⁺ cells or progeny thereof are administered by continuous infusion, or by doses at intervals of, e.g., one day, one week, or 1-7 times per week. An exemplary dose protocol is one involving the maximal dose or dose frequency that avoids significant undesirable side effects. A total weekly dose depends on the type and activity of the factors/cells being used. Determination of the appropriate dose is made by a clinician, e.g., using parameters or factors known or suspected in the art to affect treatment or predicted to affect treatment. Generally, the dose begins with an amount somewhat less than the optimum dose and is increased by small increments thereafter until the desired or optimum effect is achieved relative to any negative side effects.

The present disclosure includes the following non-limiting examples.

### Examples

### Example 1: Immunoselection of MPCs by Selection of STRO-3⁺ Cells

Bone marrow (BM) is harvested from healthy normal adult volunteers (20-35 years old). Briefly, 40 ml of BM is aspirated from the posterior iliac crest into lithium-heparin anticoagulant-containing tubes.

BMMNC are prepared by density gradient separation using Lymphoprep™ (Nycomed Pharma, Oslo, Norway) as previously described (Zannettino, A.C. et al. (1998) Blood 92: 2613-2628). Following centrifugation at 400 x g for 30 minutes at 4°C, the buffy layer is removed with a transfer pipette and washed three times in "HHF", composed of Hank's balanced salt solution (HBSS; Life Technologies, Gaithersburg, MD), containing 5% fetal calf serum (FCS, CSL Limited, Victoria, Australia).

STRO-3⁺ (or TNAP⁺) cells were subsequently isolated by magnetic activated cell sorting as previously described (Gronthos et al. (2003) Journal of Cell Science 116: 1827-1835; Gronthos, S. and Simmons, P.J. (1995) Blood 85: 929-940). Briefly, approximately 1-3 x 10⁸ BMMNC are incubated in blocking buffer, consisting of 10% (v/v) normal rabbit serum in HHF for 20 minutes on ice. The cells are incubated with 200µl of a 10µg/ml solution of STRO-3 mAb in blocking buffer for 1 hour on ice. The cells are subsequently washed twice in HHF by centrifugation at 400 x g. A 1/50 dilution of goat anti-mouse γ-biotin (Southern Biotechnology Associates, Birmingham, UK) in HHF buffer is added and the cells incubated for 1 hour on ice. Cells are washed twice in MACS buffer (Ca²⁺ - and Mn²⁺ -free PBS supplemented with 1% BSA, 5 mM EDTA and 0.01% sodium azide) as above and resuspended in a final volume of 0.9 ml MACS buffer.

One hundred µl streptavidin microbeads (Miltenyi Biotec; Bergisch Gladbach, Germany) are added to the cell suspension and incubated on ice for 15 minutes. The cell suspension is washed twice and resuspended in 0.5 ml of MACS buffer and subsequently loaded onto a mini MACS column (MS Columns, Miltenyi Biotec), and washed three times with 0.5 ml MACS buffer to retrieve the cells which did not bind the STRO-3 mAb (deposited on 19 December 2005 with American Type Culture Collection (ATCC) under accession number PTA-7282 - see International Publication No.WO 2006/108229). After addition of a further 1 ml MACS buffer, the column is removed from the magnet and the TNAP⁺ cells are isolated by positive pressure. An aliquot of cells from each fraction can be stained with streptavidin-FITC and the purity assessed by flow cytometry.

### Example 2: Cells Selected by STRO-3 mAb are STRO-1^{bright} Cells

Experiments were designed to confirm the potential of using STRO-3 mAb as a single reagent for isolating cells STRO-1^{bright} cells.

Given that STRO-3 (IgG1) is a different isotype to that of STRO-1 (IgM), the ability of STRO-3 to identify clonogenic CFU-F was assessed by two-color FACS analysis based on its co-expression with STRO-1⁺ cells isolated using the MACS procedure (Figure 1). The dot plot histogram represents 5 x 10⁴ events collected as listmode data. The vertical and horizontal lines were set to the reactivity levels of <1.0% mean fluorescence obtained with the isotype-matched control antibodies, 1B5 (IgG) and 1A6.12 (IgM) treated under the same conditions. The results demonstrate that a minor population of STRO-1^{bright} cells co-expressed TNAP (upper right quadrant) while the remaining STRO-1⁺ cells failed to react with the STRO-3 mAb. Cells isolated by FACS from all four quadrants were subsequently assayed for the incidence of CFU-F (Table 1).

**Table 1: Enrichment of human bone marrow cells by dual-color FACS analysis based on the co-expression of the cell surface markers STRO-1 and TNAP (refer to Figure 1). FACS sorted cells were cultured under standard clonogenic conditions in alpha MEM supplemented with 20% FCS. The data represents the mean number of day 14 colony-forming cells (CFU-F) per 10⁵ cells plated ± SE (n=3 different bone marrow aspirates). These data suggest that human MPC are exclusively restricted to the TNAP positive fraction of BM which co-express the STRO-1 antigen brightly.**

| Bone Marrow Fraction | Frequency of CFU-F/10⁵ Cells | Enrichment (Fold Increase) |
|---|---|---|
| Unfractionated BMMNC | 11.0 ± 2.2 | 1.0 |
| TNAP⁺/STRO-1^{bright} | 4,511 ± 185 | 410 |
| TNAP⁺/STRO-1^{dull} | 0.0 | 0.0 |

### Example 3: Relative Gene and Surface Protein Expression of STRO-1^{dull} and STRO-1^{bri} Cells

In the first series of experiments, semi-quantitative RT-PCR analysis was employed to examine the gene expression profile of various lineage-associated genes expressed by STRO-1^{dull} or STRO-1^{bri} populations, isolated by fluorescence activated cell sorting (Figure 2A). In the second series of experiments, flow cytometry and mean channel fluorescence analysis was employed to examine the surface protein expression profile of various lineage-associated proteins expressed by STRO-1^{dull} or STRO-1^{bri} populations, isolated by fluorescence activated cell sorting.

Total cellular RNA was prepared from either 2 x 10⁶ STRO-1^{bri} or STRO-1^{dull} sorted primary cells, chondrocyte pellets and other induced cultures and lysed using RNAzolB extraction method (Biotecx Lab. Inc., Houston, TX), according to the manufacturer's recommendations. RNA isolated from each subpopulation was then used as a template for cDNA synthesis, prepared using a First-strand cDNA synthesis kit (Pharmacia Biotech, Uppsala, Sweden). The expression of various transcripts was assessed by PCR amplification, using a standard protocol as described previously (Gronthos et al., J. Bone and Min. Res. 14:48-57, 1999). Primer sets used in this study are shown in Table 2. Following amplification, each reaction mixture was analyzed by 1.5% agarose gel electrophoresis, and visualized by ethidium bromide staining. RNA integrity was assessed by the expression of GAPDH.

Relative gene expression for each cell marker was assessed with reference to the expression of the house-keeping gene, GAPDH, using ImageQant software (Figure 2B, C). In addition, dual-color flow cytometric analysis was used to examine the protein expression profile of *ex vivo* expanded MPC based on their expression of a wider range of cell lineage-associated markers in combination with the STRO-1 antibody. A summary of the general phenotype based on the gene and protein expression of STRO-1^{dull} and STRO-1^{bri} cultured cells is presented in Table 3. These data indicate that *ex vivo* expanded STRO-1^{bri} MPC exhibit differentially higher expression of markers associated with perivascular cells, including angiopoietin-1, VCAM-1, SDF-1, IL-1_{β}, TNFα, and RANKL. Comparisons between the protein and gene expression profiles of STRO-1^{dull} and STRO-1^{bri} cultured cells are summarized in Tables 3 and 4.

Subtractive hybridization studies were also performed in order to identify genes uniquely expressed by STRO-1^{bri} cells. Briefly, STRO-1^{dull} and STRO-1^{bri} were isolated as described above (see Figure 3A). Total RNA was prepared from STRO-1^{dull} and STRO-1^{bri} cells pooled from 5 different marrow samples using the RNA STAT-60 system (TEL-TEST). First-strand synthesize was performed using the SMART cDNA synthesis kit (Clontech Laboratories). The resultant mRNA/single-stranded cDNA hybrid was amplified by long-distance PCR (Advantage 2 PCR kit; Clontech) using specific primer sites at the 3' and 5' prime ends formed during the initial RT process according to the manufacturer's specifications. Following RsaI digestion of the STRO-1^{bright} cDNA, 2 aliquots were used to ligate different specific adaptor oligonucleotides using the Clontech PCR-Select cDNA Subtraction Kit. Two rounds of subtractive hybridization were performed using STRO-1^{bri} (tester) and STRO-1^{dull} (driver) cDNA, and vice versa, according to the manufacturer's protocol. This procedure was also performed in reverse using STRO-1^{dull} tester cDNA hybridized against STRO-1^{bri} driver cDNA.

To identify genes uniquely expressed by STRO-1^{bri} population, STRO-1^{bri}-subtracted cDNA was used to construct replicate low-density microarray filters comprising 200 randomly selected bacterial clones transformed with the STRO-1^{bri}subtracted cDNAs ligated into a T/A cloning vector. The microarrays were subsequently probed with either [³²P] dCTP-labeled STRO-1^{bri} or STRO-1^{dull} subtracted cDNA (Figure 3B-C). Differential screening identified a total of 44 clones, which were highly differentially expressed between the STRO-1^{dull} and STRO-1^{bright} subpopulations. DNA sequencing of all the differentially expressed clones revealed that only 1 clone was representative of a known stromal cell mitogen; namely, platelet-derived growth factor (PDGF) (Gronthos and Simmons, Blood. 85: 929-940, 1995). Interestingly, 6 of the 44 clones were found to contain DNA inserts corresponding to the chemokine, stromal-derived factor-1 (SDF-1). The high abundance of SDF-1 transcripts in human STRO-1^{bright} cells was confirmed by semiquantitative RT-PCR of total RNA prepared from freshly sorted STRO-1^{bright}, STRO-1^{dull}, and STRO-1^{negative} bone marrow subpopulations (Figure 3D and Table 3).

**Table 2. RT-PCR primers and conditions for the specific amplification of human mRNA**

| **Target** | **Sense/ Antisense (5'-3') Primer Sequences** | **Produc t Size** |
|---|---|---|
| **Gene** | | |
| **GAPDH** | CACTGACACGTTGGCAGTGG (SEQ ID NO: 1) | 417 |
| | CATGGAGAAGGCTGGGGCTC (SEQ ID NO: 2) | |
| **SDF-1** | GAGACCCGCGCTCGTCCGCC (SEQ ID NO: 3) | 364 |
| | GCTGGACTCCTACTGTAAGGG (SEQ ID NO: 4) | |
| **IL-1β** | AGGAAGATGCTGGTTCCCTCTC (SEQ ID NO: 5) | 151 |
| | CAGTTCAGTGATCGTACAGGTGC (SEQ ID NO: 6) | |
| **FLT-1** | TCACTATGGAAGATCTGATTTCTTACAGT (SEQ ID NO: 7) | 380 |
| | GGTATAAATACACATGTGCTTCTAG (SEQ ID NO: 8) | |
| **TNF-α** | TCAGATCATCTTCTCGAACC (SEQ ID NO: 9) | 361 |
| | CAGATAGATGGGCTCATACC (SEQ ID NO: 10) | |
| **KDR** | TATAGATGGTGTAACCCGGA (SEQ ID NO: 11) | 450 |
| | TTTGTCACTGAGACAGCTTGG (SEQ ID NO: 12) | |
| **RANKL** | AACAGGCCTTTCAAGGAGCTG (SEQ ID NO: 13) | 538 |
| | TAAGGAGGGGTTGGAGACCTCG (SEQ ID NO: 14) | |
| **Leptin** | ATGCATTGGGAACCCTGTGC (SEQ ID NO: 15) | 492 |
| | GCACCCAGGGCTGAGGTCCA (SEQ ID NO: 16) | |
| **CBFA-1** | GTGGACGAGGCAAGAGTTTCA (SEQ ID NO: 17) | 632 |
| | TGGCAGGTAGGTGTGGTAGTG (SEQ ID NO: 18) | |
| **PPARγ2** | AACTGCGGGGAAACTTGGGAGATTCTCC (SEQ ID NO: 18) | 341 |
| | AATAATAAGGTGGAGATGCAGGCTCC (SEQ ID NO: 19) | |
| **OCN** | ATGAGAGCCCTCACACTCCTC (SEQ ID NO: 20) | 289 |
| | CGTAGAAGCGCCGATAGGC (SEQ ID NO: 21) | |
| **MyoD** | AAGCGCCATCTCTTGAGGTA (SEQ ID NO: 22) | 270 |
| | GCGAGAAACGTGAACCTAGC (SEQ ID NO: 23) | |
| **SMMHC** | CTGGGCAACGTAGTAAAACC (SEQ ID NO: 24) | 150 |
| | TATAGCTCATTGCAGCCTCG (SEQ ID NO: 25) | |
| **GFAP** | CTGTTGCCAGAGATGGAGGTT (SEQ ID NO: 26) | 370 |
| | TCATCGCTCAGGAGGTCCTT (SEQ ID NO: 27) | |
| **Nestin** | GGCAGCGTTGGAACAGAGGTTGGA (SEQ ID NO: 28) | 460 |
| | CTCTAAACTGGAGTGGTCAGGGCT (SEQ ID NO: 29) | |
| **SOX9** | CTCTGCCTGTTTGGACTTTGT (SEQ ID NO: 30) | 598 |
| | CCTTTGCTTGCCTTTTACCTC (SEQ ID NO: 31) | |
| **Collagen type X** | AGCCAGGGTTGCCAGGACCA (SEQ ID NO: 32) | 387 |
| | TTTTCCCACTCCAGGAGGGC (SEQ ID NO: 33) | |
| **Aggrecan** | CACTGTTACCGCCACTTCCC (SEQ ID NO: 34) | 184 |
| | ACCAGCGGAAGTCCCCTTCG (SEQ ID NO: 35) | |

**Table 3. Summary of the Relative Gene Expression in STRO-1^{Bri} and STRO-1^{Dull} populations. A list of genes which displayed measurable and differential expression between the STRO-1^{Bri} and STRO-1^{Dull} populations as determined by reverse transcription-PCR are presented . Values represent the relative gene expression with reference to the house-keeping gene, GAPDH.**

| | | **Gene Expression relative to GAPDH** | |
|---|---|---|---|
| **Tissue** | **Marker** | **STRO-1^{Bri}** | **STRO-1^{Dull}** |
| **Glial Cells (within Neural Tissue)** | *GFAP (Glial Fibrillary Acidic Protein)* | 0.1 | 0.7 |
| **Bone** | *OCN (Osteocalcin)* | 1.1 | 2.5 |
| | *OSX (Osterix)* | 0.4 | 1.3 |
| | *CBFA-1 (Core Factor Binding Protein-1)* | 0.3 | 0.6 |
| **Immunoregulatory** | *RANKL (Receptor Activator of Nuclear Factor κ B)* | 1.6 | 0.3 |
| **Neuroprotective/ neuro-chemoattractant** | *SDF-1-alpha (Stromal Derived factor-1-alpha)* | 3.2 | 0.1 |
| **Fat** | *Leptin* | 3.1 | 4.2 |
| **Cardiomyocytes** | *GATA-4* | 1.1 | 2.9 |
| **Endothelial cells** | *Ang-1 (Angiopoietin-1)* | 1.5 | 0.8 |
| **Chondrocytes** | *Sox 9* | 0.3 | 1.1 |
| | *COL X (Collagen X)* | 3.5 | 2.8 |
| **Pro-inflammatory Cytokines** | *TNF-alpha (Tumor necrosis alpha)* | 1.7 | 0.9 |

To correlate protein surface expression with density of STRO-1 expression, single cell suspensions of *ex vivo* expanded cells derived bone marrow MPC were prepared by trypsin/EDTA detachment and subsequently incubated with the STRO-1 antibody in combination with antibodies identifying a wide range of cell lineage-associated markers. STRO-1 was identified using a goat anti-murine IgM-fluorescein isothiocyanate while all other markers were identified using either a goat anti-mouse or anti-rabbit IgG- phycoerythrin. For those antibodies identifying intracellular antigens, cell preparations were first labeled with the STRO-1 antibody, fixed with cold 70% ethanol to permeabilize the cellular membrane and then incubated with intracellular antigen-specific antibodies. Isotype matched control antibodies were used under identical conditions. Dual-colour flow cytometric analysis was performed using a COULTER EPICS flow cytometer and list mode data collected. The dot plots represent 5,000 listmode events indicating the level of fluorescence intensity for each lineage cell marker (y-axis) and STRO-1 (x-axis). The vertical and horizontal quadrants were established with reference to the isotype matched negative control antibodies.

**Table 4. Summary of the Relative Protein Expression in STRO-1^{Bri} and STRO-1^{Dull} populations. A list of proteins which displayed differential expression between the STRO-1^{Bri} and STRO-1^{Dull} populations as determined by flow cytometry are presented. Values represent the relative mean fluorescence intensity of staining.**

| | | **Mean Fluorescence Intensity** | |
|---|---|---|---|
| **Tissue** | **Marker** | **STRO-1^{Bri}** | **STRO-1^{Dull}** |
| **Neurons** | *Neurofilament* | 1.7 | 20.5 |
| **Bone** | *ALK PHOS (Alkaline Phophatase)* | 5.7 | 44.5 |
| **Immunoregulatory** | *RANKL (Receptor Activator of Nuclear Factor κ B)* | 658.5 | 31.0 |
| **Epithelial Cells** | *CytoKeratin 10*+*13* | 1.2 | 23.3 |
| | *Cytokeratin 14* | 1.8 | 8.8 |
| **Smooth Muscle** | *α-SMA (Alpha Smooth Muscle Actin* ) | 318.0 | 286.0 |
| **Chondrocytes** | *Byglycan* | 84.4 | 65.9 |
| **Basal Fibroblast** | *Tenascin C* | 22.2 | 6.9 |
| **Cardiomyocyte** | *Troponin C* | 2.5 | 15.0 |

### Example 4: human dental pulp stem cells (hDPSC) differentiate into neurons

### Isolating DPSCs and Human Foreskin Fibroblasts

DPSCs and human exfoliated deciduous teeth (SHEDs) were isolated essentially as described by Gronthos et al., Proc Natl Acad Sci U.S.A 97:13625-13630, 2000. Briefly, discarded normal human impacted third molars were collected from adults (19-35 years of age) or exfoliated teeth (7-8 years of age) with informed consent of patients undergoing routine extractions at the Dental Clinic of the University of Adelaide, under approved guidelines set by the University of Adelaide and Institute of Medical and Veterinary Science Human Subjects Research Committees (H-73-2003). Tooth surfaces were cleaned and cracked open using a vice to reveal the pulp chamber. The pulp tissue was gently separated from the crown and root and then digested in a solution of 3 mg/ml collagenase type I and 4 mg/ml dispase for 1 hour at 37°C. Single-cell suspensions were obtained by passing the cells through a 70-µM strainer. Cultures were established by seeding single-cell suspensions (1-2 × 10⁵) of dental pulp into T-25 flasks in growth media, (a-modification of Eagle's medium supplemented with 20% fetal calf serum, 100 µM 1-ascorbic acid 2-phosphate, 2 mM 1-glutamine, 100 U/ml penicillin, and 100 µg/ml streptomycin), then incubated at 37°C in 5% CO2.

Human foreskin fibroblasts (HFFs) were isolated by collagenase/dispase digestion from explants of foreskin biopsies from neonatal male donors, with informed parental consent.

### Neural Induction Assay

Tissue culture flasks or wells were coated with polyornithine (10 µg/ml final concentration) overnight at room temperature. Flasks and wells were washed twice with water and then coated with laminin (5 µg/ml final concentration) overnight at 37°C in a humid incubator. Flasks and wells were washed with phosphate-buffered saline (PBS), and then with growth media before cells were added. Cells were thawed and grown in T25-coated flasks until 80% confluent. Cells were reseeded at a density of 1.5 × 105/well in 2 ml/well growth media, for 3 days in 6-well coated plates, or 2 × 103 cells in 8-well coated chamber slides. Cells were then cultured in either media A or media B for 3 weeks. Media A consists of Neurobasal A Media (10888-022; Invitrogen, Carlsbad, CA, USA), 100 U/ml penicillin, 1× B27 supplement, 100 µg/ml streptomycin, 20 ng/ml epidermal growth factor (EGF, 100-15; PeproTech, Rocky Hill, NJ, USA), and 40 ng/ml basic fibroblast growth factor (FGF, no. 104FGFB01; Prospec Tany Techno Gene, Rehovot, Israel). Media B comprises three separate media conditions for the duration of the 3-week period: the first incubation was media A for 7 days, followed by a change in media consisting of 50:50 ratio of Dulbecco's modified Eagle's medium (DMEM, 12100-046; Invitrogen) and F12 media (21700-075; Invitrogen), and insulin-transferrin-sodium-selenite supplement (ITTS, 11074547001; Roche Diagnostics, Basel, Switzerland), 100 U/ml penicillin, 100 µg/ml streptomycin, and 40 ng/ml FGF for 7 days. The final 7 days of incubation consisted of media containing 50:50 ratio of DMEM and F12 media, ITTS, 100 U/ml penicillin and 100 µg/ml streptomycin, 40 ng/ml FGF, and 0.5 µM retinoic acid (RA, R2625; Sigma-Aldrich, St. Louis, USA). Control samples were maintained in growth media for the duration of the neuronal inductive assay. The media for all conditions were replaced twice weekly.

### Electrophysiology

At the completion of the 3-week neuronal differentiation assay, cells were liberated with trypsin and replated onto glass coverslips treated with hydrochloric acid at a concentration of 2 × 10⁴ cells/500 µL in neuronal differentiation media or growth media and incubated for 24 hours. Whole-cell patch clamping was performed at room temperature using a computer-based patch clamp amplifier (EPC-9; HEKA Electronics, Lambrecht/Pfalz, Germany) and PULSE software (HEKA Electronics). The bath solution contained (in mM): NaCl, 140; KCl, 4; CaCl2, 2; MgCl2, 2; glucose, 10; and HEPES, 10; adjusted to pH 7.4 with NaOH. To block K⁺ channels and separate Na⁺ currents, K glutamate and KCl in the internal solution were replaced with equimolar amounts of Cs glutamate and CsCl. Patch pipettes were pulled from borosilicate glass and fire polished; pipette resistance ranged between 2-4 MΩ. All voltages shown were corrected for liquid junction potential (-14 mV and -18 mV for K⁺- and Cs⁺-based internal solutions, correspondingly), estimated by JPCalc (provided by Dr. P.H. Barry, University of New South Wales, Sydney, Australia, 1994). The holding potential was -90 mV throughout.

### Avian Embryo Injections

Chicken eggs (white leghorn; HICHICK Breeding Company, Bethal, Australia) were placed longways in egg trays and incubated in a humidified incubator at 37°C for approximately 40 hours to reach stages 10-12 prior to injection. Green fluorescent protein (GFP)-transduced adult human DPSCs or HFFs (5 × 10³ cells/µL) were injected into the developing avian embryo. Fast green dye (2 µL) was added to the cells to visualize them during the injection procedure. Embryos were prepared by wiping the egg with 70% ethanol, albumin was extracted from the egg using a needle, a window was cut into the top of the egg, and the embryo was visualized by injecting Indian ink (Winsor and Newton, Middlesex, England) (1:10 prepared in Ringer's solution [7.2 g NaCl₂, 0.17 g CaCl₂, 0.37 g KCl, 0.115 g Na₂HPO₄ in 1 L ddH₂O, adjusted to pH 7.4, filter sterilized]) underneath the embryo. Ringer's solution was placed on top of the embryo to maintain moisture. The vitelline membrane was removed from around the head of the embryo using tungsten wire. The cells were placed in a glass capillary needle (GC100TF-10; SDR Clinical Technology, Sydney, Australia,), attached to a micromanipulator and pressure injector (Narishage, Tokyo), set at 25 volts. The micromanipulator was used to guide the needle into the region directly adjacent to rhombomere 2 in the developing hindbrain. Cells were injected into the embryo using a foot pump attached to the pressure injector. The glass needle was removed and a few drops of Ringer's solution were placed on top of the embryo. The egg was sealed with sticky tape and placed back in the humidified incubator. Following the incubation period, embryos were cut out of the egg and placed in cold PBS; GFP-injected cells were visualized using a fluorescent dissecting microscope. Embryos were then dissected; the head was cut as an open book, i.e., cut from the nose toward the hindbrain down the length of the head, along the ventral side. Dissected embryos were fixed in 4% PFA for either 2 hours at room temperature or overnight at 4°C, and then washed twice with PBS prior to immunofluorescent staining.

### Immunocytochemistry of Cultured Cell Preparations

Chamber slides were fixed with 4% PFA for 30 minutes at room temperature and then washed with PBS and 0.1% Tween 20 (Sigma-Aldrich) (PBS-T). Cultures were blocked (10% horse serum in PBS-T) for 30 minutes at room temperature and then incubated with primary antibody (1:100 Nestin (611658; BD Biosciences, San Diego, USA); 1:500 polysialic acid-neural cell adhesion molecule (PSA-NCAM) (gift from Dr. T. Seki), 1:500 β-III tubulin clone, TUJ1 (MMS-435P, 1 mg/ml; Covance, Princeton, NJ, USA); 1:200 NF-M (13-0700, 0.5 mg/ml; Zymed, South San Francisco, CA); 1:500 NF-H (AB1991; Chemicon, Temecula, CA, USA) in blocking solution overnight at 4°C. Mouse, goat, and rabbit (Ig) controls were treated under the same conditions. After washing, the secondary antibodies (1:200 goat anti-mouse Alexa 488 [A11029, 1.5 mg/ml; Jackson Immunoresearch Laboratories, West Grove, PA, USA]; 1:200 goat anti-rabbit Alexa 488 [A11034, 2 mg/ml [Molecular Probes Inc., Eugene, OR, USA]) were added in blocking solution for 2 hours at room temperature in the dark. Finally, the slides were washed and coverslipped with Prolong gold antifade with 4',6-diamidino-2-phenylindole dihydrochloride (DAPI, P36931; Invitrogen).

### Results

As shown in Figure 4, when hDPSC are grown in a neuronal inductive media they differentiate into multiprocess neuronal-like cells with the biophysical property to conduct a sodium current. Furthermore, when hDPSC are transplanted into the brain of a chick embryo they differentiate into neuronal-like cells which express neurofilament.

### Example 4: hDPSC treatment improves forelimb motor function post-stroke in a rodent model

The therapeutic efficacy of hDPSC was assessed in a rat model of middle cerebral artery occlusion (MCAO) which results in ischemic stroke. MCAO is the most common presentation of ischemic stroke in humans and this pre-clinical rodent model of MCAO recapitulates the human condition. In this model, MCAO is created by inserting a nylon filament into the internal carotid artery which is pushed forward to occlude the middle cerebral artery, which lies distal to the internal carotid artery.

### Methods

### Human DPSC Culture and Preparation

Human DPSCs were isolated from adult impacted (failed to emerge fully into its expected position) third molars by digestion of pulp tissue and retrovirally transduced with the fluorescence marker green fluorescent protein (GFP). DPSCs at passages 3-5 were cryopreserved in 10% dimethyl sulfoxide in fetal calf serum. Cells were thawed and passaged at least once prior to use for intracerebral transplantation. DPSCs were cultured in modified Eagle's medium (Sigma-Aldrich, St. Louis, http://www.sigmaaldrich.com) supplemented with 20% fetal calf serum, 100 ┐ M L-ascorbic acid 2-phosphate (Wako, Richmond, VA), 2 mM L-glutamine, 100 U/ml penicillin, and 100 µg/ml streptomycin at 37°C in 5% CO₂. Cells were dissociated with trypsin and resuspended in modified Eagle's medium without supplements at a final concentration of 1.5 x 10⁵ cells per microliter. Cell viability was determined by the trypan blue dye exclusion method.

### Rodent Middle Cerebral Artery Occlusion and Intracerebral Transplantation

Fifty-four adult male Sprague-Dawley rats weighing 300±20 g were maintained on a 12-hour light/dark cycle with ad libitum access to food and water.

Rats were anesthetized with isoflurane and subjected to temporary, focal cerebral ischemia via intraluminal proximal middle cerebral artery occlusion (MCAo) [essentially as described in Aspey et al., Neuropathol Appl Neurobiol., 26: 232-242, 2000 and Spratt et al., JNeurosci Methods, 155: 285-290, 2006]. Rectal temperature Temperature was maintained at 37 ± 1°C by a heating pad, and blood oxygen saturation and pulse rate were monitored and maintained within physiological parameters with a pulse oximeter (model 8500AV; Nonin Medical, Plymouth, MN). In brief, the right common carotid artery, external carotid artery (ECA), internal carotid artery (ICA), and pterygopalatine artery were exposed through blunt dissection, followed by cauterization and ligation of the ECA. A 4-0 monofilament nylon thread (Dynek, Hendon, SA, Australia) with a silicone-coated tip (diameter 0.35 mm, length 5 mm) was advanced from the ECA stump, through the ICA, and up to the origin of the right middle cerebral artery (MCA). The thread was secured in place, and the incision was closed. After 2 hours of occlusion, rats were reanesthetized, and the thread was withdrawn to allow reperfusion. Rats were allowed to recover from surgery and were assessed for contralateral forelimb flexion by briefly suspending the animal by the tail and for circling behavior on the floor to verify MCAo at 2 hours. At 24 hours post-MCAo, animals were randomly selected to receive human DPSC (n = 31) or vehicle (n = 23) treatment (see Figure 5 depicting the treatment schedule). A total volume of 4µl cell suspension (6x10⁵ cells) was injected (1 µL/minute) into the ipsilateral parenchyma at two stereotaxic coordinates (anterior-posterior, medial-lateral relative to bregma; dorsal-ventral from dura): 2 µl into the striatum (-0.40, +4.00; -5.50 mm) and another 2µl into the cortex (-0.40, +4.00; -1.75 mm) using a stereotaxic frame (Kopf Instruments, Tujunga, CA). Vehicle animals were treated similarly, with the administration of α-modified Eagle's medium as the vehicle. All animals received a daily subcutaneous injection of 10 mg/kg cyclosporin A
(Novartis, Basel, Switzerland).

### 2,3,5-Triphenyltetrazolium Chloride Staining

2,3,5-Triphenyltetrazolium chloride (TTC) staining was performed to confirm ischemia. Fresh brains were cut into 2-mm slices and incubated in 2% TTC (Sigma-Aldrich) at 37°C for 10 minutes in the dark. Brain sections were color imaged using a LiDE210 flatbed scanner (Canon, Tokyo).

### Functional Assessment

Animals were trained for 3 consecutive days prior to MCAo, and the third session (day 1) was used as the preoperative baseline. Blinded assessment of behavior was performed during the light cycle at days 1 (prior to DPSC transplantation), 7, 14, 21, and 28 post-MCAo (three trials in each test) (Fig. 1C). Animals that failed pre-MCAo neurobehavioral training (in that the animals did not achieve baseline scores in each test) and exhibited either few to none or very severe functional deficits at least 1 week following MCAo were excluded. The experiments were undertaken with random treatment assignment and blinded outcome assessment, which conformed to Stroke Therapy Academic Industry Roundtable (STAIR) and similar guidelines.

### Neuroscore

Post-MCAo gross neurological dysfunction for each animal was evaluated using a scoring system modified from previous studies [Barry et al., Brain Res, 1389: 143-151, 2011; Bederson Stroke, 17: 472-476, 1986; and Thal et al., J. Neurol. Sci, 286: 150-159, 2008]. Higher scores indicate greater neurological impairment.

### Adhesive Tape Removal Test

Adhesive labels (15 mm2) were applied on the medial aspect of the rats' forepaws [Bouet et al., Nat Protoc 4: 1560-1564, 2009]. The order of sticky label placement onto the contralateral or ipsilateral forepaw was randomized. Upon return to the testing cage, the time spent attempting to remove the adhesive label and the total time used to remove the label (allocated maximum of 2 minutes) from each paw were recorded. The duration spent removing (or attempting to remove) the label was expressed as a percentage of the total time taken.

### Step Test

Each rat was held firmly using one hand to slightly raise the rat's torso and hind limbs and the other hand to restrain one of the forepaws, so as to bear weight on the other forepaw that was not constrained [Schallert et al., Exp Neurol, 64: 33-43, 1979]. The rat was then moved sideways (30 cm) so that the weight-bearing forepaw was stepping in the backhand direction to adjust for body movement. The order of forepaw testing was randomized. The number of steps performed with each forelimb was recorded.

### Rotarod

Rats were placed on a rotarod device consisting of a motorized rotating ensemble of 18 x 1-mm rods [28]. Animals were required to walk on the apparatus as the rotational speed was accelerated from 0 to 24 rpm within 2 minutes. The time at which the animals completed the trial, fell off the device, or gripped the rungs and spun for two consecutive rotations without attempting to walk on the rungs was recorded.

### Ledged/Tapered Beam Walking

Animals were required to traverse an elevated beam that was tapered along its extent into a darkened box at the end [Schallert T, Woodlee MT. Orienting and placing. In: Whishaw IQ, Kolb B, eds. The Behavior of the Laboratory Rat: A Handbook with Tests. Oxford, U.K.: Oxford University Press 2005;129 -140], and their performance was videotaped. The number of foot slips made with each hind limb onto an underhanging ledge (2 cm below the upper beam surface) was calculated as a percentage of the total number of steps taken to traverse the beam. Beam walking could not be completed at day 1 post-MCAo because of the circling behavior of stroke animals.

### Immunohistochemistry and Imaging

At the conclusion of neurobehavioral assessment, rats were deeply anesthetized, injected with 1,000 U of heparin directly into the heart, and transcardially perfused with ice-cold saline followed by 4% (wt/vol) paraformaldehyde in phosphate-buffered saline (PBS). Brains were removed and postfixed in the same fixative at 4°C overnight. For colorimetric detection of surviving human DPSCs, brains were embedded in paraffin and cut into serial 5-µmcoronal sections. Sections were blocked with 3% normal horse serum in PBS and washed twice in PBS, before incubation with mouse antihuman mitochondria (1:1,500; Millipore, Billerica), followed by biotinylated anti-mouse IgG (1:250; Vector Laboratories, Burlingame, CA) and by streptavidin horseradish peroxidaseconjugated antibody (1:1,000; Thermo Scientific, Rockford, IL). Antibodies were diluted in blocking solution. Antibody staining was visualized with 3,3-diaminobenzidine (Sigma-Aldrich). Bright-field images were acquired and analyzed using the NanoZoomer Digital Pathology System (Hamamatsu Photonics, Shizuoka, Japan). The number of grafted cells remaining in the rodent brain (n = 3) at 4 weeks post-transplantation was counted in every second 5-µmcoronal section, and the distribution of grafted cells was analyzed.

For double immunofluorescence studies, 100-µmfree-floating sections were blocked with 10% normal horse serum in PBS containing 0.3% Triton X-100, before human DPSCs were identified using goat anti-GFP (1:250; Rockland, Gilbertsville, PA), mature neurons using mouse anti-neuronal nuclei (NeuN; 1:500; Millipore) or astrocytes using rabbit anti-glial fibrillary acidic protein (GFAP; 1:250; DAKO, Glostrup, Denmark). Other primary antibodies used were mouse anti-β-III tubulin (1:500; Millipore), mouse anti-nestin (1:100; Abcam, Cambridge, U.K.), mouse anti-polysialic acid-neural cell adhesion molecule (PSA-NCAM; 1:200; Millipore) and mouse anti-O4 (1: 500; Sigma-Aldrich). Secondary antibodies were either Cy2-conjugated anti-goat IgG, Cy3-conjugated anti-rabbit IgG, or Cy3-conjugated anti-mouse IgG (1:300; Jackson Immunoresearch Laboratories, West Grove, PA). Antibodies were diluted in blocking solution. All sections were mounted with ProLong Gold antifade reagent (Invitrogen, Carlsbad, CA). To estimate the percentage of GFP-positive cells that coexpressed NeuN or GFAP, z-stack images at a magnification of┐ 40 were taken in five fields, which consisted of two cortical and three striatal regions in the ipsilateral hemisphere at the level of DPSC transplantation (n=3 animals). Confocal images were acquired sequentially using a TCS SP5 confocal microscope and were processed using its LAS AF software (Leica Microsystems, Wetzlar). Orthogonal projections were performed using ImageJ (NIH).

### Measurement of Corpus Callosum Thickness

Paraffin sections, at the levels of bregma and bregma±0.1 mm, were stained with 0.1% cresyl violet (n = 6 DPSC-treated, n = 2 vehicle-treated). Measurements of corpus callosum thickness were made at the midline (midpoint between the two hemispheres) and at the lateral edge of the ventricles in both hemispheres of the brain. Measurements were expressed as a percentage of corresponding midline measurements and were done using an AxioVision imaging software (Carl Zeiss, Oberkochen, Germany).

### Statistical Analysis

Analysis was carried out using GenStat (13th edition; VSN International, Hemel Hempstead, U.K.). The effect of treatment on behavioral test scores was analyzed using repeated-measures analysis of variance (ANOVA), with a Greenhouse-Geisser correction to account for correlation between successive measurements of an individual rat, and with adjustments for prestroke data as covariates to allow for possible differences in the initial ability of the rats. A post hoc Fisher's protected least significant difference (LSD) test was carried out if the initial ANOVA was significant. Differences were considered statistically significant at p<0.05. Data were presented as box plots using R (version 2.10.1; R Development Core Team, Vienna, Austria) or as bar graphs using Prism (version 5.02, GraphPad Software, Inc., San Diego). Reported values are mean ± SEM.

### Results

### Transplanted Human DPSCs Enhanced Poststroke Forelimb Sensorimotor Recovery

The ability of human DPSC treatment following focal cerebral ischemia to improve neurobehavioral function over time was investigated. No deleterious effects following DPSC treatment were observed in comparison with controls in the regards to morbidity or mortality rates. Mortality rates obtained following intracerebral transplantation were comparable (approximately 9%) in both treatment groups.

The clinically relevant rodent model of unilateral MCAo causes substantial ischemic damage to the dorsolateral striatum and the overlying frontal/parietal cortex in the ipsilateral hemisphere of the rodent brain. As a result, animals developed neurobehavioral deficits demonstrated by an increase in neuroscore of 11.7 ± 0.4 points at day 1 poststroke (Figure 6A). In addition, marked forelimb somatosensory and motor asymmetries were exhibited in the step test (Figure 6B; a decrease of 82.2 ± 6.6% in the number of steps taken by the contralateral forelimb at day 1 post-MCAo) and the adhesive tape removal test (Figure 6C; a decrease of 93.7±2.8% in time attempting to remove the external stimulus from the contralateral forepaw at day 1 post-MCAo).

Upon experimental stroke, loss of motor balance and coordination was also observed in the rotarod test (Figure 6D; a decrease of 53.0 ± 2.0% in time spent on the rotarod at day 1 post-MCAo).

Stroke-affected animals showed hind limb asymmetry (Figure 6E, an increase of 21.7 ± 6.2%) in contralesional hind limb footslips during beam walking at 1 week post-MCAo. The ipsilateral limbs had normal poststroke neurobehavioral function.

All animals exhibited gradual recovery in neurobehavioral function over 4 weeks poststroke (Figure 6). DPSC-treated animals demonstrated significantly enhanced global neurobehavioral function (Figure 6A; p < 0.018 group x day interaction, repeated measures ANOVA) and significantly lower neuroscores in comparison with the vehicle-treated group, specifically at 21 (p < 0.05, post hoc Fisher's protected LSD) and 28 (p<0.01) days poststroke. In limb-specific neurobehavioral studies, DPSC-treated animals demonstrated significantly enhanced contralateral (stroke-affected) forelimb sensorimotor recovery in comparison with vehicle controls in the step test (Figure 6B; p <0.045 overall group effect) and the adhesive tape removal test (Fig. 2C; p <0.049 overall group effect). Taken together, DPSC-treated animals showed reduced poststroke deficits in the use of the contralateral forelimb at all days measured.

There was no significant difference in ipsilateral limb function between treatment groups. Performances in the rotarod (Figure 6D) and the tapered/ledged beam walking (Figure 6E) tests were not statistically significantly different (p < 0.05) between treatment groups. One possible explanation for the difference in enhanced functional improvement between the limbs is that this rodent MCAo model produces more destruction to the sensorimotor cortical representation for forelimb function in the rodent brain because of preferential vascular supply to this region [Salo et al., Soc Neurosc Abstr 13: 1268, 1987]. In support of this, in the animals in the present study there was a severe loss of forelimb function as measured by the step and adhesive tape removal tests, but a less dramatic impairment of hind limb function as evaluated by the tapered/ledged beam walking test immediately post-MCAo. Rotarod running and beam walking assess motor coordination and hind limb function, respectively [Hicks et al., Cell stem Cell, 5: 139-140, 2009], and these tests might not be sensitive enough to detect poststroke deficits. The enhanced functional improvement following DPSC treatment in comparison with vehicle-treated animals became evident over the 4 weeks of analysis of forelimb but not hind limb function.

An alternative explanation may relate to learned compensatory motor behaviors in the animal. In the rotarod running and tapered/ledged beam walking tests, through repeated testing, rodents may have learned novel ways in which stroke-unaffected limbs or tail deviation can be used to compensate for loss of function in the stroke-affected limb [Schaar et al., Exp Transl Stroke Med, 2: 13, 2010]. Forelimb-specific tasks allowed minimal use of compensatory behaviors to overcome poststroke loss of sensorimotor function, as measured by the step and adhesive tape removal tests.

The foregoing data indicate that hDPSC transplantation influenced the normal functioning of the host nervous system following stroke and resulted in improved neurological function compared to control animals. Without being bound by theory or mode of action, this improvement may have occurred through neuroplastic change within the brain following stroke. For example, as shown above STRO-1⁺ cells express SDF-1 (CXCL-12). hDPSCs have also been shown to express this chemokine. Moreover, hDPSC chemoattract axons from the host trigeminal ganglion following transplantation into the head of a chick embryo, even though these animals demonstrated a significant functional improvement at the later time points.

### Survival of DPSCs in Ischemic Brain at 4 Weeks Post-Transplantation

The findings from the neurobehavioral studies following DPSC transplantation indicated peak benefit at later time points in neurological scores and forelimb sensorimotor function. Subsequent studies investigated whether the underlying cellular mechanism to explain these findings was due to neural cell replacement following DPSC transplantation into the stroke brain. For example, the number of donor cells that had survived at the end point of the study when peak benefit was observed was determined. Individual DPSCs within the rodent brain were visualized using a specific antibody against human mitochondrial antigen, which does not cross-react with rat tissue. The number of labeled cells was counted manually in every second coronal brain section, that is, at 10-µm intervals. The total number of human mitochondria-positive cells detected in the brain of each animal at 4 weeks after transplantation ranged from 9,458 to 22,394 cells (Table 5). This gave an average number of 13,807 - 4,293 remaining human DPSCs and a mean survival rate of 2.3±0.7% of the originally transplanted 6x10⁵ cells. Our data demonstrated that cell transplantation was successful and resulted in long-term viability, albeit at low numbers, of DPSC-derived cells in the postischemic brain.

Without being bound by theory or mode of action, since only a mean of 2.3% of the initially transplanted hDPSC survive to 28 days post-stroke, neuronal replacement is unlikely to be the dominant mechanism of action by which hDPSC improves neurological function. However, such neuronal replacement may provide some therapeutic benefit. Alternative or additional mechanisms that may be responsible for this improvement include neuroprotection, angiogenesis, immune-modulation and neuroplasticity.

**Table 5: Survival of transplanted human DPSCs at 4 weeks**

| | Rat 1 | Rat 2 | Rat 3 | Mean ± SEM |
|---|---|---|---|---|
| No. of surviving human DPSCs | 9,458 | 9,570 | 22,394 | 13,807 ± 4,293 |
| DPSC survival rate {%} | 1.6 | 1.6 | 3.7 | 2.3 ± 0.7 |

| | | | | |
|---|---|---|---|---|
| Total number and percentage of surviving human DPSCs (taken as a percentage of 600,000 cells originally transplanted) at 4 weeks after middle cerebral artery occlusion. Serial 5-*µ*m rat brain coronal sections were stained for human mitochondrial antigen to demonstrate human DPSCs, and human mitochondrial antigen-positive cells were counted manually in every other section. Abbreviation: DPSC, dental pulp stem cell. | | | | |

### Targeted Migration of DPSCs Toward the Stroke Lesion

The spatial distribution of human DPSCs following transplantation into the ischemic rat brain was also analyzed. At 24 hours post-transplantation, DPSCs were found clustered around both injection sites. After 4 weeks, human mitochondria-labeled cells were found predominantly in the area surrounding the infarct core (Figures 7, 8A, 8B), demonstrating that transplanted cells had migrated from the sites of transplantation preferentially toward the ischemic infarct and localized at the ischemic boundary zone (IBZ). A small number of DPSCs were observed in the hemisphere contralateral to the site of cell transplantation (Figures 7, 8A, 8B) and in the corpus callosum (Figure 8B). Cells had migrated at least 4 mm into the contralateral hemisphere over the 4-week period. DPSC-derived cells were widely distributed in the contralateral hemisphere at low numbers as compared with the ipsilesional hemisphere, and there was no obvious migration of DPSCs into specific targeted regions of the contralateral hemisphere. On rare occasions DPSC-derived cells were incorporated into and around the walls of cerebral microvessels with morphological appearances consistent with endothelial cells (Figure 4C, arrows), pericytes, or smooth muscle cells (Figure 4C, arrowhead).

### Transplanted Human DPSCs Differentiated into Neurons and Astrocytes In Vivo

Morphologies of human mitochondrial antigen-positive cells in the 4-week postischemic brain were then examined. The cells appeared to have morphologies consistent with astrocytic cells that demonstrated a stellar-like appearance (Figure 8D, arrows), whereas presumptive neuronal cells exhibited long processes (Figure 8D, arrowhead). To verify these phenotypes, double immunofluorescence analysis and confocal imaging were used. Transplanted human DPSCs had previously been retrovirally transduced to express GFP, which allowed colabeling with either NeuN or GFAP to identify mature neurons or astrocytes, respectively. Colocalization of GFAP with GFP expression in cells within the glial scar confirmed that DPSC-derived cells had differentiated into astrocytes. Within the ipsilateral peri-infaret cortical region, NeuN and GFP double-positive cells indicated differentiation of transplanted DPSCs into neurons. The distribution of astrocytes and neurons in the postischemic ipsilateral cerebral hemisphere was not uniform. At the IBZ in the striatal region that DPSC-derived astrocytes predominated (51.0±8.6% GFAP/GFP double-positive cells, expressed as a percentage of GFP single-labeled cells), whereas in comparison, there was a smaller proportion of DPSC-derived neurons (8.7 ± 6.1% NeuN/GFP cells). In the peri-infarct cortical region, the proportion of astrocytes and neurons appeared similar (38 ± 0.6% GFAP/GFP cells and 32.1 ± 1.8% NeuN/GFP cells respectively). Transplanted DPSCs found along the IBZ in the striatum predominantly differentiated into astrocytes, which appeared to be incorporated into the glial scar. There were more DPSC-derived neurons present in the cortical peri-infarct region as compared with the striatum. Of interest, approximately one-third of GFP cells in the peri-infarct cortical region were also labeled with the early/intermediate neuronal marker β-III tubulin. Within the glial scar along the IBZ, however, many GFP cells also co-expressed the neural progenitor marker nestin. There was little evidence of nestin/GFP cells in the cortex. A few GFP cells were also positive for the migratory neuroblast marker PSA-NCAM in the peri-infarct cortex. On rare events DPSC-derived oligodendrocytes (O4/GFP cells) were observed in the rodent brain at 4 weeks post-transplantation.

### DPSC Treatment Reduced Poststroke Corpus Callosum Atrophy

The effect of DPSC treatment on the loss of interhemispheric connectivity across the corpus callosum following a stroke insult was also investigated. At 4 weeks post-stroke, there was a nonsignificant decrease in ipsilateral corpus callosum atrophy in DPSC-treated animals in comparison with the vehicle-treated group (DPSC-treated, 67.6 ± 12.5%, L2 measurements expressed as a percentage of corresponding midline L1 measurements; vehicle-treated, 56.6 ± 3.0%; p > .05) (Figure 9). In DPSC-treated brains, the thickness of the corpus callosum in the ipsilateral hemisphere showed a trend toward better preservation and attained a similar corpus callosum thickness of the contralesional (stroke-unaffected) hemisphere. There was no difference in corpus callosum thickness in the contralateral hemisphere between treatment groups (DPSC-treated, 66.1 ± 14.0%; vehicle-treated, 66.5 ± 1.6%).

### Example 5: Characterization of Cynomolgus Monkey STRO-3⁺ MPCs

Simian marrow progenitor cells (from cynomolgus monkeys; cyno-MPC) were isolated from ∼15 ml of bone marrow aspirate collected from a female *Macaca fascicularis*. The marrow aspirate suspension was separated using a Ficoll gradient and washed to remove non-nucleated cells (red blood cells). The nucleated cells were counted then separated by attaching CA12 antibody (anti-STRO-3) and Dynalbeads. The cells with antibody and beads attached were positively selected by the magnetic field of an MPC-1 magnet. The positive selected cells were counted and seeded into T-flasks at passage (p.) 0 in Growth Medium. Pre-selection, positive, and negative cells were used in a colony forming assay (CFU-F).

The cyno-MPC cells were fed with Growth Media. All cultures (p.0 - p.5) were fed every 2 to 4 days until they reached desired confluence. The cells were then passaged or harvested using HBSS wash and then collagenase followed by Trypsin/Versene. The p.1 cells were counted and seeded into T-flasks. When the p.1 cyno-MPC reached desired confluence the cells were harvested and cryopreserved using a controlled rate freezer.

Passage 1 cryopreserved cyno-MPC were thawed and seeded into T-flasks (p.2). The p.2 cells were passaged into a Cell Factory at p.3. The p.3 cells were harvested and passaged to p.4 in to a Cell Factory. Extra p.3 cells were cryopreserved. The p.4 cells were passaged to 6 x Cell Factories at p.5. When the p.5 cyno-MPC reached desired confluence the cells were harvested and cryopreserved using a controlled rate freezer. The cells were cryopreserved in 50% AlphaMEM, 42.5% Profreeze, and 7.5% DMSO. Samples were tested for CFU-F assay, FACS, sterility, mycoplasma, and endotoxin.

Results of representative flow cytometry analysis of the immunophenotype of cultured cyno-MPCs are shown in Figure 10. As shown, these cells are STRO-1⁺, STRO-4⁺ and CD146⁺.

### Example 6 Prospective Isolation of Non Human Primate (nhp)DPSC:

The use of magnetic bead sorting allows for the purification of rare DPSC from a fibrous pulp tissue. Following dental pulp tissue harvest and enzymatic digestion, approximately 1-2 x 10⁵ cells can be recovered from one adult human third molar. In view of this, pooled cells derived from multiple teeth are collected from the same animal. Single cell suspensions of non-human primate dental pulp are incubated with antibodies directed against human MPC-associated antigens that we have been previously shown to react with nonhuman primate bone marrow cells (Figure 10), including STRO-1 (mouse IgM anti-human MPC; Developmental Studies Hybridoma Bank, University of Iowa, Iowa City, IA, USA); CD146 (mouse IgG2a anti-human CD146/MUC-18; 9); or STRO-4 (mouse IgG1 anti-human heat shock protein-β). Labeled pulp cells are incubated with either sheep anti-mouse IgG-conjugated or rat anti-mouse IgM-conjugated magnetic Dynabeads (4 beads per cell: Dynal, Oslo, Norway). Magnetic bead-positive cells are collected using the MPC-1 magnetic particle concentrator(Dynal) according to the manufactures recommended protocol. The STRO-1, STRO-4 or CD146 bead positive cells are seeded at about 3 x 10⁴ cells per cm² in growth medium to generate primary cultures in normal growth media (a-MEM supplemented with 20% (v/v) FBS, 2 mM L-glutamine, 100 mM L-ascorbate-2-phosphate, 50 U/ml penicillin, 50 mg/ml streptomycin). After reaching about 90% confluence, primary DPSC will be subcultured at a plating density of about 0.5-1 x 10⁴ cells per cm².

### Example 7 Assessment of nhpDPSC Colony-Frequency: Using Colony Forming-Fibroblast Assays

The efficiency of the STRO-1, STRO-4 or CD146 antibodies as single reagents to purify nhpDPSC is assessed. Unfractionated and STRO-1, STRO-4 or CD146 positive and negative selected cell fractions are seeded into 6-well culture plates at 0.3, 1.0 and 3.0 x 10⁴ cells per well in normal growth media, in triplicate and incubated at 37°C in 5% CO₂ and >90% humidity for 12 days. Aggregates of greater than ≥50 cells are scored as colony-forming units-fibroblastic (CFU-F). The antibody selection protocol that results in the highest incidence of CFU-F colony formation is used to generate GLP-grade nhpDPSC for the functional characterization studies.

### Example 8 Characterisation of nhpDPSC

Flow cytometric analysis is to characterize the immunophenotype of *ex vivo* expanded DPSC. The expression of mesenchymal and non-mesenchymal stem-cell associated surface markers at different cell passages is assessed as described above. As allogeneic nhpDPSC are used to treat ischemic stroke, immunomodulatory capacity of theses cells to suppress activated immune cells when co-cultured in a mixed lymphocyte reaction or with mitogen stimulated lymphocytes in vitro is assessed (e.g., as described in Wada et al., J Cell Physiol., 219: 667-676, 2009).

The capacity of DPSC to generate mesodermal and ectodermal tissues including those similar to from which they are derived, is a hallmark feature of these cells. The ability of DPSC to differentiate into different cell lineages *in vitro* is investigated by culturing under inductive conditions. *Ex vivo* expanded nhpDPSC are used to assess the capacity for multi-lineage differentiation into osteoblasts (Alizarin red staining of mineral deposits), adipocytes (Oil red O staining of lipid droplets) and chondrocytes (Toluidine Blue staining of sulphated proteoglycans and collagen type II expression) e.g., as described in Grontos et al., J Cell Sci., 116: 1827-2835, 2003.

Single cell suspensions of *ex vivo* expanded nhpDPSC pre-labelled with GFP, are additionally transplanted subcutaneously into the dorsal surface of severe compromised immuno-deficient (NOD/SCID) mice (n=3) for a period of about eight weeks in combination with ceramic hydroxyapatite/tricalcium phosphate (HA/TCP) particles as a carrier vehicle. Following eight weeks post-transplantation, the recipient animals will be sacrificed to retrieve the transplants for histological analysis.

Assessment of *in vitro* differentiation of nhpDPSC into neural cells is performed in chamber slides coated with polyornithine under neural-inductive culture conditions as described above.

Real-time PCR and immunohistochemistry is used to assess the gene and protein expression of early (NeuroD, Nestin, NeuN, b-III tubulin) and late (GFAP, Neurofilament medium and heavy chains).

Patch clamping is performed to determine the differentiated nhpDPSC capacity to initiate an action potential *in vitro.*

In addition, neurogenic and neuroplastic potential of GFP-labelled nhpDPSC is determined by injecting these cells into chick embryos *in ovo*, e.g., as described above.

The electrophysiological properties of implanted GFP-labelled DPSC are assessed by measuring the presence of voltage-dependent sodium and potassium channels on 300mm sections of brain tissue at 7 and 14 days post engraftment.

Collectively, these studies define the allogeneic nhpDPSC population that is used to treat stroke in a non-human primate pre-clinical study.

### Example 9: Use of nhpDPSC to Improve Motor Function in a Non-Human Primate Models of Ischemic Stroke

The similarities between human and nonhuman primates, with respect to complex cognitive capabilities, great social complexity and intricacy of brain organization make them good models to study neurological disease and disorders.

A non-human primate model of ischemic stroke is produced a MCAO method using nylon filament obstruction via the internal carotid artery (e.g., as described in Freret et al., J Cereb Blood Flow Metab., 28: 786-796, 2008). The non-human primate model is produced in cynomolgus macaques, an Old World monkey (*Macaca fascicularis*). The following is a program of the experimental design for the ischemic stroke study:
(i) Baseline neuro-behavioral testing is performed on all animals prior to stroke.
(ii) MCAO model of ischemic stroke is induced.
   In brief, the monkey are anaesthetized, intubated and mechanically ventilated with intensive monitoring of blood oxygen levels, core temperature, blood pressure, ECG and laser-Doppler flowmetry of the intracranial blood flow. Detailed surgery is performed by an experienced neuroscientist to expose the right common carotid artery at the bifurcation of the external and internal carotid arteries. The external carotid artery and other branching arteries are cauterized to ensure smooth passage of the filament along the internal carotid artery to the origin of the middle cerebral artery. A 0.54 mm diameter by 3mm long silicone rubber-coated nylon filament is inserted into the internal carotid artery and advanced until resistance is felt indicating that the filament has occluded the middle cerebral artery. The filament is held in place by a ligature. Three hours post-MCAO the silicone rubber-coated nylon filament is withdrawn which results in reperfusion, a process which approximates middle cerebral artery ischemic stroke in humans.
(iii) MRI is carried out within 7 days post-MCAO to determine if an ischemic stroke, of appropriate size and site, has resulted.
(iv) The cell therapy design comprises three groups with intra-striatal injection of 5x10⁵ or 1.5x10⁶ nhpDPSC or media alone (without cells) at one week post-stroke. As in rodents, there is a limit as to the volume of fluid that can be safely injected into the cerebrum post-stroke due to morbidity from raised intracranial pressure. This is estimated to be 12 µL in the macaque. Thus, all intrastriatal injections will be of 12 µL with varying cell concentrations. The monkey is anaesthetized as before and a burr-hole made in the skull for stereotaxic intra-striatal injection.
(iv) Neuro-behavioral testing is undertaken on a weekly basis for the 6-week duration of the study. To adequately investigate the extent of sensori-motor deficit following MCA ischemic stroke the following tests are used: Monkey CANTAB® (CAmbridge Neurophysiological Test Automated Battery), Neuroscore, Hill-and-Valley Staircase and Tactile forelimb stimulation tests (e.g., as described in Bihel et al., J Cereb Blood Flow Metab., 30: 273-285, 2010).
(vi) Study completed at the 6th week following stroke.

### Example 10: Interplay Between Engrafted nhpDPSC and NHP Brain Tissue

### 10.1 Rescue of the ischemic penumbra:

Around the ischemic stroke there is a region of ischemic penumbral tissue that is at risk of death but may be rescued. STRO-1⁺ cell therapy may interact with this ischemic penumbra and thus limit the stroke size.

Monkey are anaesthetized and positioned Prone in a .0T Siemens Trio scanner and T2-W turbo-spin echo images (T2WI, TR/TE=4800/86 ms) are acquired with a field of view (180 x 180 mm) and image acquisition matrix of 0.5 x 0.5 in plane resolution. Twelve T2WI coronal images are acquired with a slice thickness of 2 mm gapped at 0.2 mm. The infarct volume on T2WI will be delineated using Siemens MRI software for each monkey.

### 10.2 Neuronal replacement and connectivity in NHP stroke brain

Infarction results in loss of neurons and glia in approximately 2/3 of one cerebral hemisphere. The aim of this series of investigations is to determine: (1) the number of nhpDPSC that survive, (2) the number of nhpDPSC that differentiate into neurons or glia, and (3) whether nhpDPSC-derived neurons make functional connections. To do this, nhpDPSC are retrovirally transduced to express GFP and cultured, prior to transplantation, in BrdU (about 20 µM for 24 hours) for nuclei labeling. These two labeling methods provide formal identification of the engrafted nhpDPSC within the host brain.

The number of nhpDPSC that have differentiated into a neural phenotype are stereologically estimated by establishing the density of nhpDPSC in a specified volume. From the rodent study we have found that at 4 weeks, hDPSC migrate into the site of the stroke to approximately 1 mm in either antero-posterior direction from the site of the injection (which is evident from the scar on the surface of the brain). Thus, an 8 mm thickness of monkey brain in an antero-posterior axis to the injection site includes the majority of transplanted human cells by 6 weeks post-stroke. 100 mm serial coronal sections along this axis are taken (∼ 4x103mm tissue width @ 100 mm = 40 coronal sections per treated animal). A random sampling protocol (using Image J) is constructed to count the number of BrdU-positive cells in a standardized tissue volume. Direct 3-dimensional counting of positively stained human cells is undertaken using a Bio-Rad MRC-1000UV Confocal Microscope (Adelaide Microscopy). Confocal microscopy identifies GFP-positive or BrdU-positive nhpDPSC to be co-localized with cytoplasmic antigens expressed by differentiated cells (e.g. neurons using antibodies against neurofilament medium chain (NF-M) or β-III tubulin, astocytes expressing glial fibrillary acidic protein, GFAP or Tyrosine Hydroxylase).

Electrophysiological properties of nhpDPSC-derived neurons engrafted into the monkey stroke are also assessed. Firstly, the biophysical properties of single nhpDPSC-derived neurons isolated from the rodent stroke brain are assessed. Briefly, the animals are decapitated and the brain removed and placed in ice-cold oxygenated saline. Brain slices 300-500 mm thick are cut using a vibrating tissue slicer, washed and then enzymatically digested by a mixture of trypsin and collagenase. Single neurons are obtained by gentle pipetting of the tissue, washed and plated on collagen-treated glass coverslips. Following 24-hour recovery, cells are used for whole-cell patch clamping. nhpDPSC-derived neurons are identified as GFP-positive cells. Appropriate voltage protocols and channel inhibitors are used to identify the types of voltage-gated channels expressed by these cells.

The ability of nhpDPSC-derived neurons to generate action potentials (APs) and to form connections with other neurons is investigated by patch clamping and imaging of cells in brain slices. Brain slices 250-400 mm thick are cut on a vibratome in proximity to the site of injection. After a period of 1-hour recovery at room temperature and constant oxygenation, individual slices are transferred into a recording chamber mounted on an upright BX51WI Olympus microscope with fluorescence attachment, IR and DIC optics. The nhpDPSC are identified first using GFP fluorescence and then visualized using IR filter (720-850 nm) and Rolera-XR IR-sensitive camera. Whole-cell patch clamping of neurons in brain slices is performed using an EPC-10 computer-controlled amplifier and associated equipment. APs are evoked by injecting depolarizing current and recorded in current clamp mode.

To investigate whether APs generated by nhpDPSC can propagate to the surrounding neurons through either tight junctions or synaptic connections, Fura-2 fluorescence is used to visualize changes of intracellular Ca2⁺ concentration in conjunction with patch clamping. Briefly, a brain slice is loaded with membrane permeable Fura-2AM, washed and placed into the recording chamber. Engrafted nhpDPSC-derived neurons are identified by GFP fluorescence and patch clamped using IR. The fluorescence setting is then changed to those appropriate for Fura-2 measurements. The APs are evoked in nhpDPSC by passing depolarizing current and fluorescence is measured in the surrounding rodent host neurons.

### 10.3 Functional MRI (fMRI) and Diffusion Tensor Imaging (DTI) of non-human primate stroke brains to investigate neuroplasticity:

These technologies allow non-invasive investigation of neuroplasticity following nhpDPSC treatment as compared to control monkeys. In the ischemic stroke model, fMRI is used to assess forelimb motor control and determine if there is a greater activation of the motor cortex following nhpDPSC treatment. The fMRI studies are undertaken at 3 and 6 weeks following treatments.

## Claims

1. A population of cells enriched for non-hematopoietic STRO-1⁺ multipotential cells for use in regenerating cerebral neurons in a subject who has suffered a stroke, wherein the population of cells is to be administered between 24 hours and 1 month after the stroke.

2. The population of cells for use according to claim 1, wherein the cerebral neurons are in the cerebral cortex.

3. The population of cells for use according to claim 2, wherein the cerebral neurons are in the motor cortex and/or sensory cortex.

4. The population of cells for use according to claim 1, which is for improving cerebral function or preventing loss of cerebral function in the subject who has suffered a stroke.

5. The population of cells for use according to claim 1, which is for treating or preventing a movement disorder in the subject who has suffered a stroke.

6. The population of cells for use according to any one of claims 1 to 5, wherein the stroke is an ischemic stroke.

7. The population of cells for use according to any one of claims 1 to 6, wherein the population of cells is to be administered between 48 hours and 1 month after the stroke.

8. The population of cells for use according to claim 7, wherein the population of cells is to be administered between 72 hours and 1 month after the stroke.

9. The population of cells for use according to any one of claims 1 to 8, wherein the population of cells is enriched for STRO-1^{bright} cells.

10. The population of cells for use according to any one of claims 1 to 9, wherein the population of cells is derived from bone marrow or dental pulp.

11. The population of cells for use according to any one of claims 1 to 10, wherein the population of cells is to be administered systemically or locally to the brain of the subject.

12. The population of cells for use according to any one of claims 1 to 11, wherein the population of cells is to be administered a plurality of times.

13. The population of cells for use according to any one of claims 1 to 12, wherein between 0.1 x 10⁶ to 5 x 10⁶ STRO-1⁺ cells per kg are to be administered or between 0.3 x 10⁶ to 2 x 10⁶ STRO-1⁺ cells per kg are to be administered or between 0.1 x 10⁵ and 0.5 x 10⁶ STRO-1⁺ cells per kg are to be administered.

14. The population of cells for use according to any one of claims 1 to 13, wherein the population of cells has been culture expanded prior to administration.

15. The population of cells for use according to any one of claims 1 to 6, wherein the population of cells is to be administered between 48 hours and 2 weeks after the stroke.

## Patentansprüche

1. Zellpopulation, die für nicht-hämatopoetische STRO-1⁺ multipotente Zellen angereichert ist, zur Verwendung bei der Regeneration von zerebralen Neuronen in einem Lebewesen, das einen Schlaganfall erlitten hat, wobei die Zellpopulation zwischen 24 Stunden und 1 Monat nach dem Schlaganfall zu verabreichen ist.

2. Zellpopulation zur Verwendung nach Anspruch 1, wobei sich die zerebralen Neuronen in der Großhirnrinde befinden.

3. Zellpopulation zur Verwendung nach Anspruch 2, wobei sich die zerebralen Neuronen im motorischen Kortex und/oder sensorischen Kortex befinden.

4. Zellpopulation zur Verwendung nach Anspruch 1, die zur Verbesserung der Gehirnfunktion oder zur Verhinderung des Verlustes der Gehirnfunktion bei dem Lebewesen, das einen Schlaganfall erlitten hat, dient.

5. Zellpopulation zur Verwendung nach Anspruch 1, die zur Behandlung oder Vorbeugung einer Bewegungsstörung bei dem Lebewesen, das einen Schlaganfall erlitten hat, dient.

6. Zellpopulation zur Verwendung nach einem der Ansprüche 1 bis 5, wobei der Schlaganfall ein ischämischer Schlaganfall ist.

7. Zellpopulation zur Verwendung nach einem der Ansprüche 1 bis 6, wobei die Zellpopulation zwischen 48 Stunden und 1 Monat nach dem Schlaganfall zu verabreichen ist.

8. Zellpopulation zur Verwendung nach Anspruch 7, wobei die Zellpopulation zwischen 72 Stunden und 1 Monat nach dem Schlaganfall zu verabreichen ist.

9. Zellpopulation zur Verwendung nach einem der Ansprüche 1 bis 8, wobei die Zellpopulation für STRO-1^{bright} Zellen angereichert ist.

10. Zellpopulation zur Verwendung nach einem der Ansprüche 1 bis 9, wobei die Zellpopulation von Knochenmark oder Zahnpulpa abgeleitet ist.

11. Zellpopulation zur Verwendung nach einem der Ansprüche 1 bis 10, wobei die Zellpopulation systemisch oder lokal an das Gehirn des Lebewesens zu verabreichen ist.

12. Zellpopulation zur Verwendung nach einem der Ansprüche 1 bis 11, wobei die Zellpopulation mehrmals verabreicht werden soll.

13. Zellpopulation zur Verwendung nach einem der Ansprüche 1 bis 12, wobei zwischen 0,1 x 10⁶ bis 5 x 10⁶ STRO-1⁺ Zellen pro kg zu verabreichen sind oder zwischen 0,3 x 10⁶ bis 2 x 10⁶ STRO-1⁺ Zellen pro kg zu verabreichen sind oder zwischen 0,1 x 10⁵ und 0,5 x 10⁶ STRO-1⁺ Zellen pro kg zu verabreichen sind.

14. Zellpopulation zur Verwendung nach einem der Ansprüche 1 bis 13, wobei die Zellpopulation vor der Verabreichung in Kultur vermehrt wurde.

15. Zellpopulation zur Verwendung nach einem der Ansprüche 1 bis 6, wobei die Zellpopulation zwischen 48 Stunden und 2 Wochen nach dem Schlaganfall zu verabreichen ist.

## Revendications

1. Population de cellules enrichie en cellules multipotentes STRO-1⁺ non hématopoïétiques pour une utilisation dans la régénération de neurones cérébraux chez un sujet qui a souffert d'un accident vasculaire cérébral, laquelle population de cellules est destinée à être administrée entre 24 heures et 1 mois après l'accident vasculaire cérébral.

2. Population de cellules pour une utilisation selon la revendication 1, dans laquelle les neurones cérébraux sont dans le cortex cérébral.

3. Population de cellules pour une utilisation selon la revendication 2, dans laquelle les neurones cérébraux sont dans le cortex moteur et/ou le cortex sensoriel.

4. Population de cellules pour une utilisation selon la revendication 1, qui est destinée à améliorer la fonction cérébrale ou à empêcher une perte de fonction cérébrale chez le sujet qui a souffert d'un accident vasculaire cérébral.

5. Population de cellules pour une utilisation selon la revendication 1, qui est destinée à traiter ou à prévenir un trouble du mouvement chez le sujet qui a souffert d'un accident vasculaire cérébral.

6. Population de cellules pour une utilisation selon l'une quelconque des revendications 1 à 5, dans laquelle l'accident vasculaire cérébral est un accident ischémique cérébral.

7. Population de cellules pour une utilisation selon l'une quelconque des revendications 1 à 6, laquelle population de cellules est destinée à être administrée entre 48 heures et 1 mois après l'accident vasculaire cérébral.

8. Population de cellules pour une utilisation selon la revendication 7, laquelle population de cellules est destinée à être administrée entre 72 heures et 1 mois après l'accident vasculaire cérébral.

9. Population de cellules pour une utilisation selon l'une quelconque des revendications 1 à 8, laquelle population de cellules est enrichie en cellules STRO-1^{bright}.

10. Population de cellules pour une utilisation selon l'une quelconque des revendications 1 à 9, laquelle population de cellules dérive de moelle osseuse ou de pulpe dentaire.

11. Population de cellules pour une utilisation selon l'une quelconque des revendications 1 à 10, laquelle population de cellules est destinée à être administrée par voie systémique ou locale au cerveau du sujet.

12. Population de cellules pour une utilisation selon l'une quelconque des revendications 1 à 11, laquelle population de cellules est destinée à être administrée plusieurs fois.

13. Population de cellules pour une utilisation selon l'une quelconque des revendications 1 à 12, dans laquelle entre 0,1 x 10⁶ et 5 x 10⁶ cellules STRO-1⁺ par kg sont destinées à être administrées, ou entre 0,3 x 10⁶ et 2 x 10⁶ cellules STRO-1⁺ par kg sont destinées à être administrées, ou entre 0,1 x 10⁵ et 0,5 x 10⁶ cellules STRO-1⁺ par kg sont destinées à être administrées.

14. Population de cellules pour une utilisation selon l'une quelconque des revendications 1 à 13, laquelle population de cellules a été développée par culture avant l'administration.

15. Population de cellules pour une utilisation selon l'une quelconque des revendications 1 à 6, laquelle population de cellules est destinée à être administrée entre 48 heures et 2 semaines après l'accident vasculaire cérébral.
